# EUROPEAN PATENT APPLICATION

(11) **EP 3 939 568 A1**
(43) Date of publication of application: **19.01.2022**
(21) Application number: 21188208.9
(22) Date of filing: 19.02.2020
(51) Int. Cl.: A61K 9/00, A61N 1/00, A61P 35/00, A61P 31/00, A61K 31/513, A61K 31/555, A61K 31/7068

(54) **TREATING GASTRIC CANCER USING TTFIELDS AND DRUG IN COMBINATION**

(30) Priority: 22.02.2019 US 201962808923 P; 08.10.2019 US 201962912483 P; 08.11.2019 US 201962932834 P
(62) Divisional of application: 20708665.3
(71) Applicant: Novocure GmbH, 6039 Root D4 (CH)
(72) Inventor: GILADI, Moshe, 40691 Moshav Herut (IL); GOTLIB, Karnit, 390809 Tirat Carmel (IL); SHNAIDERMAN, Rosa, 35012 Haifa (IL); WEINBERG, Uri, 3055707 Binyamina (IL); ZEEVI, Einav, 3091666 Zichron Yaakov (IL)
(74) Representative: Boden, Keith McMurray

(57) **Abstract**

Treating gastric cancer or reducing the viability of gastric cancer cells in a subject or reducing the viability of gastric cancer cells by administering fluorouracil to the subject or the cancer cells, and applying an alternating electric field to a target region of the subject or the cancer cells are provided.

## Description

### BACKGROUND

Despite a worldwide decline in incidence, gastric cancer remains the fourth most common cancer (incidence of approximately 1,000,000/year) and the second most common cause of cancer death worldwide (approximately 750,000 per year). Despite improvement of systemic therapy in the recent era, long-term survival rates for patients with advanced gastric cancer remains poor.

XELOX is a chemotherapy regimen made up of oxaliplatin and capecitabine. FOLFOX is the standard treatment today for gastric cancer. FOLFOX is a chemotherapy regimen for treatment of gastric cancer, made up of three drugs: Folinic acid (Leucovorin); Fluorouracil (5-FU); and Oxaliplatin.

Tumor Treating Fields (TTFields) are an effective anti-neoplastic treatment modality delivered via application of low intensity, intermediate frequency, alternating electric fields. TTFields therapy has received FDA approval for treating Glioblastoma Multiforme brain tumors. TTFields therapy is delivered using a wearable and portable device (Optune^{®}). The delivery system includes four adhesive, non-invasive, insulated "transducer arrays", an electric field generator, rechargeable batteries, and a carrying case. The transducer arrays are applied to the skin in the vicinity of the tumor and are connected to the field generator.

In the preclinical setting, TTFields can be applied in vitro using the Inovitro^{™} system by means of perpendicular pairs of transducer arrays insulated by a high dielectric constant ceramic. Inovitro^{™} (TTFields lab bench system) is comprised of a TTFields generator and base plate containing 8 ceramic dishes per plate.

### SUMMARY OF THE INVENTION

Methods of treating gastric cancer in a subject by administering oxaliplatin and capecitabine or folinic acid, fluorouracil, and oxaliplatin to the subject and applying an alternating electric field to a target region of the subject are provided. The alternating electric field has a frequency between 100 and 400 kHz.

In some instances, methods of treating gastric cancer in a subject by administering oxaliplatin and capecitabine or folinic acid, fluorouracil, and oxaliplatin to the subject, and applying an alternating electric field to a target region of the subject are provided. The alternating electric field has a frequency between 100 and 400 kHz.

In some instances, methods of reducing viability of gastric cancer cells by administering oxaliplatin and capecitabine or folinic acid, fluorouracil, and oxaliplatin to the cancer cells, and applying an alternating electric field to the cancer cells are provided. The alternating electric field has a frequency between 100 and 400 kHz.

In some instances, methods of treating gastric cancer in a subject by administering fluorouracil to the subject, and applying an alternating electric field to a target region of the subject are provided. The alternating electric field has a frequency between 100 and 400 kHz.

In some instances, methods of reducing viability of gastric cancer cells by administering fluorouracil to the cancer cells, and applying an alternating electric field to the cancer cells are provided. The alternating electric field has a frequency between 100 and 400 kHz.

In some instances, methods of reducing viability of gastric cancer cells in a subject by administering fluorouracil to the subject, and applying an alternating electric field to the gastric cancer cells in the subject are provided. The alternating electric field has a frequency between 100 and 400 kHz.

### BRIEF DESCRIPTION OF THE DRAWINGS

Figure 1 shows the exemplary cytotoxic effect of oxaliplatin with and without TTFields in AGS cells;
Figure 2 shows exemplary cell apoptosis analysis using 7AAD/AnnexinV staining of AGS cells treated with oxaliplatin with and without TTFields;
Figure 3 shows the exemplary clonogenic effect of oxaliplatin with and without TTFields in AGS cells;
Figure 4 shows the overall effect of oxaliplatin with and without TTFields in AGS cells;
Figure 5 shows the exemplary cytotoxicity effect of 5-FU with and without TTFields in AGS cells;
Figure 6 shows the results of an exemplary cell apoptosis analysis using 7AAD/AnnexinV staining of AGS cells treated with 5-FU with and without TTFields;
Figure 7 shows the exemplary clonogenic effect of 5-FU with and without TTFields in AGS cells;
Figure 8 shows the exemplary overall effect of 5-FU with and without TTFields in AGS cells;
Figure 9 shows the exemplary cytotoxicity effect of leucovorin with and without TTFields in AGS cells;
Figure 10 shows an exemplary apoptosis analysis (percent of cell fraction of 7AAD and AnnexinV staining) for each leucovorin concentration;
Figure 11 shows the exemplary clonogenic effect of leucovorin with and without TTFields in AGS cells;
Figure 12 shows the exemplary overall effect of leucovorin with and without TTFields in AGS cells;
Figure 13 shows the exemplary cytotoxicity effect of each chemotherapy alone and FOLFOX with and without TTFields in AGS cells (^{∗∗∗}P<0.001, ^{∗∗}P <0.01, ^{∗} P<0.05);
Figure 14 shows an exemplary cell apoptosis analysis using 7AAD/AnnexinV staining of AGS cells treated with chemotherapy with and without TTFields;
Figure 15 shows the exemplary clonogenic effect of each chemotherapy alone and FOLFOX with and without TTFields in AGS cells (^{∗∗∗}P<0.001, ^{∗∗} P<0.01);
Figure 16 shows an exemplary overall effect of each chemotherapy alone and FOLFOX with and without TTFields in AGS cells (^{∗∗∗}P<0.001, ^{∗∗} P<0.01, ^{∗} P<0.05);
Figure 17 shows the exemplary cytotoxicity effect of oxaliplatin with and without TTFields in KATO III cells;
Figure 18 shows an exemplary cell apoptosis analysis using 7AAD/AnnexinV staining of KATO III cells treated with oxaliplatin with and without TTFields;
Figure 19 shows the exemplary clonogenic effect of oxaliplatin with and without TTFields in KATO III cells;
Figure 20 shows an exemplary overall effect of oxaliplatin with and without TTFields in KATO III cells;
Figure 21 shows the exemplary cytotoxicity effect of 5-FU with and without TTFields in KATO III cells;
Figure 22 shows an exemplary cell apoptosis analysis using 7AAD/AnnexinV staining of KATO III cells treated with 5-FU with and without TTFields;
Figure 23 shows the exemplary clonogenic effect of 5-FU with and without TTFields in KATO III cells;
Figure 24 shows the exemplary overall effect of 5-FU with and without TTFields in KATO III cells;
Figure 25 shows the exemplary cytotoxicity effect of leucovorin with and without TTFields in KATO III cells;
Figure 26 shows an exemplary apoptosis analysis (percent of cell fraction of 7AAD and AnnexinV staining) of each leucovorin concentration;
Figure 27 shows an exemplary clonogenic effect of Leucovorin with and without TTFields in KATO III cells;
Figure 28 shows an exemplary overall effect of leucovorin with and without TTFields in KATO III cells;
Figure 29 shows an exemplary cytotoxicity effect of each chemotherapy alone and FOLFOX with and without TTFields in KATO III cells (^{∗∗∗}P<0.001, ^{∗∗} P<0.01);
Figure 30 shows an exemplary apoptosis analysis using 7AAD/AnnexinV staining of KATO III cells treated with chemotherapy with and without TTFields;
Figure 31 shows an exemplary clonogenic effect of each chemotherapy alone and FOLFOX with and without TTFields in KATO III cells (^{∗∗∗}P<0.001); and
Figure 32 shows an exemplary overall effect of each chemotherapy alone and FOLFOX with and without TTFields in KATO III cells. (^{∗∗∗}P<0.001).

### DESCRIPTION OF THE PREFERRED EMBODIMENTS

Aspects described herein are directed to the use of tumor treating fields ("TTFields") in combination with one or more of oxaliplatin, 5-fluorouracil ("5-FU"), leucovorin (also known as folinic acid), and capecitabine to treat cancer (e.g., gastric cancer).

In another aspect, TTFields treatment can be combined with oxaliplatin, 5-FU, leucovorin (also referred to herein as "FOLFOX") to treat cancer. In yet another aspect, TTFields treatment can be combined with oxaliplatin and capecitabine (also referred to herein as "XELOX") to treat cancer.

Gastric cancer may be treated with a combination of TTFields and folinic acid, fluorouracil, and oxaliplatin. Gastric cancer may also be treated with a combination of TTFields and fluorouracil. The term "treating" refers to ameliorating, inhibiting, reducing growth, inhibiting metastases, and prescribing medication to do the same.

One aspect of the invention is directed to a method of treating gastric cancer in a subject. This aspect comprises administering oxaliplatin and capecitabine to the subject and applying an alternating electric field to a target region of the subject, the alternating electric field having a frequency between 100 and 400 kHz.

In some instances, at least a portion of the applying is performed after the administering and before the oxaliplatin and capecitabine ("drugs") are eliminated from the subject's body. The term "eliminated from the subject's body" refers to, for example, (1) metabolism of one or more of the drugs in the liver or elsewhere in the body resulting in excretion of substantially all of one or more of the drugs from the body, or (2) reducing the concentration of the one or more of the drugs in the bloodstream of the subject such that the drugs no longer provide substantially all of their therapeutic effect. The term "substantially" as used herein refers to greater than about 50, 60, 70, 80, 90, or 100%.

In some instances, the applying has a duration of at least 72 hours. The application of the electrical field for 72 hours may be accomplished in a single 72 hour interval. Alternatively, the application of the electrical field could be interrupted by breaks. For example, 6 sessions with a duration of 12 hours each, with a 2 hour break between sessions. The term "break" refers to an interval of time where the electrical field is not applied.

In some instances, the frequency of the alternating electric field is between 120 and 180 kHz. In some instances, the frequency of the alternating electric field is between 145 and 155 kHz.

In some instances, the oxaliplatin and capecitabine are administered at therapeutically effective doses, and the alternating electric field has a field strength of at least 1 V/cm in at least a portion of the target region. In some of these instances, the applying has a duration of at least 72 hours and the frequency of the alternating electric field is between 120 and 180 kHz. In some of these instances, the applying has a duration of at least 72 hours and the frequency of the alternating electric field is between 145 and 155 kHz.

Another aspect of the invention is directed to a method of reducing viability of gastric cancer cells. This aspect comprises administering oxaliplatin and capecitabine to the cancer cells; and applying an alternating electric field to the cancer cells, the alternating electric field having a frequency between 100 and 400 kHz. The term "reducing viability," as used herein, refers to decreasing proliferation, inducing apoptosis, or killing cancer cells.

In some instances, at least a portion of the applying is performed simultaneously with at least a portion of the administering.

In some instances, the applying has a duration of at least 72 hours. The application of the electrical field for 72 hours may be accomplished in a single 72 hour interval. Alternatively, the application of the electrical field could be interrupted by breaks. For example, 6 sessions with a duration of 12 hours each, with a 2 hour break between sessions.

In some instances, the frequency of the alternating electric field is between 120 and 180 kHz. In some instances, the frequency of the alternating electric field is between 145 and 155 kHz.

In some instances, each of the oxaliplatin and capecitabine is delivered to the cancer cells at a therapeutically effective concentration, and the alternating electric field has a field strength of at least 1 V/cm in at least some of the cancer cells. In some of these instances, the applying has a duration of at least 72 hours and the frequency of the alternating electric field is between 120 and 180 kHz. In some of these instances, the applying has a duration of at least 72 hours and the frequency of the alternating electric field is between 145 and 155 kHz. The term "therapeutically effective concentration," as used herein, refers to a concentration of a drug or drugs sufficient to achieve its intended purpose (e.g., treatment of cancer).

One aspect of the invention is directed to a method of treating gastric cancer in a subject. This aspect comprises administering folinic acid, fluorouracil, and oxaliplatin to the subject and applying an alternating electric field to a target region of the subject, the alternating electric field having a frequency between 100 and 400 kHz.

In some instances, at least a portion of the applying is performed after the administering and before the folinic acid, fluorouracil, and oxaliplatin ("drugs") are eliminated from the subject's body. The term "eliminated from the subject's body" refers to, for example, (1) metabolism of one or more of the drugs in the liver or elsewhere in the body resulting in excretion of substantially all of one or more of the drugs from the body, or (2) reducing the concentration of the one or more of the drugs in the bloodstream of the subject such that the drugs no longer provide substantially all of their therapeutic effect. The term "substantially" as used herein refers to greater than about 50, 60, 70, 80, 90, or 100%.

In some instances, the applying has a duration of at least 72 hours. The application of the electrical field for 72 hours may be accomplished in a single 72 hour interval. Alternatively, the application of the electrical field could be interrupted by breaks. For example, 6 sessions with a duration of 12 hours each, with a 2 hour break between sessions. The term "break" refers to an interval of time where the electrical field is not applied.

In some instances, the frequency of the alternating electric field is between 120 and 180 kHz. In some instances, the frequency of the alternating electric field is between 145 and 155 kHz.

In some instances, the folinic acid, fluorouracil, and oxaliplatin are administered at therapeutically effective doses, and the alternating electric field has a field strength of at least 1 V/cm in at least a portion of the target region. In some of these instances, the applying has a duration of at least 72 hours and the frequency of the alternating electric field is between 120 and 180 kHz. In some of these instances, the applying has a duration of at least 72 hours and the frequency of the alternating electric field is between 145 and 155 kHz.

Another aspect of the invention is directed to a method of reducing viability of gastric cancer cells. This aspect comprises administering folinic acid, fluorouracil, and oxaliplatin to the cancer cells; and applying an alternating electric field to the cancer cells, the alternating electric field having a frequency between 100 and 400 kHz. The term "reducing viability," as used herein, refers to decreasing proliferation, inducing apoptosis, or killing cancer cells.

In some instances, at least a portion of the applying is performed simultaneously with at least a portion of the administering.

In some instances, the applying has a duration of at least 72 hours. The application of the electrical field for 72 hours may be accomplished in a single 72 hour interval. Alternatively, the application of the electrical field could be interrupted by breaks. For example, 6 sessions with a duration of 12 hours each, with a 2 hour break between sessions.

In some instances, the frequency of the alternating electric field is between 120 and 180 kHz. In some instances, the frequency of the alternating electric field is between 145 and 155 kHz.

In some instances, each of the folinic acid, fluorouracil, and oxaliplatin is delivered to the cancer cells at a therapeutically effective concentration, and the alternating electric field has a field strength of at least 1 V/cm in at least some of the cancer cells. In some of these instances, the applying has a duration of at least 72 hours and the frequency of the alternating electric field is between 120 and 180 kHz. In some of these instances, the applying has a duration of at least 72 hours and the frequency of the alternating electric field is between 145 and 155 kHz. The term "therapeutically effective concentration," as used herein, refers to a concentration of a drug or drugs sufficient to achieve its intended purpose (e.g., treatment of cancer).

Another aspect of the invention is directed to a method of treating gastric cancer in a subject. This aspect comprises administering fluorouracil to the subject; and applying an alternating electric field to a target region of the subject, the alternating electric field having a frequency between 100 and 400 kHz.

In some instances, at least a portion of the applying is performed after the administering and before the fluorouracil is eliminated from the subject's body or exhausted. In some instances, the applying has a duration of at least 72 hours. The application of the electrical field for 72 hours may be accomplished in a single 72 hour interval. Alternatively, the application of the electrical field could be interrupted by breaks. For example, 6 sessions with a duration of 12 hours each, with a 2 hour break between sessions.

In some instances, the frequency of the alternating electric field is between 120 and 180 kHz. In some instances, the frequency of the alternating electric field is between 145 and 155 kHz.

In some instances, the fluorouracil is administered at therapeutically effective doses, and the alternating electric field has a field strength of at least 1 V/cm in at least a portion of the target region. In some of these instances, the applying has a duration of at least 72 hours and the frequency of the alternating electric field is between 120 and 180 kHz. In some of these instances, the applying has a duration of at least 72 hours and the frequency of the alternating electric field is between 145 and 155 kHz.

In some instances, the administration of the fluorouracil is accomplished by direct administration of the fluorouracil to the subject. In some instances, the administration of the fluorouracil is accomplished by indirect administration of the fluorouracil to the subject. The indirect administration of the fluorouracil can be accomplished, for example, by administering a pre-prodrug (e.g., capecitabine) to the subject, wherein the pre-prodrug is converted to fluorouracil in the subject's body.

The term "indirectly" or "indirect," as used herein, refers to administering a precursor, pre-prodrug, prodrug, or a different drug to a subject that is converted to fluorouracil in the body (e.g., the liver). See, e.g., Schellens, The Oncologist, 2007;12:152― 15; Hanneke et al., Cancer Research, 63, 7609 ―7612, November 15, 2003. In some instances, administering a precursor, pre-prodrug, prodrug, or a different drug to a subject can be advantageous by reducing side effects or altering pharmacokinetics in a desirable manner.

Another aspect of the invention is directed to a method of reducing viability of gastric cancer cells. This aspect comprises administering fluorouracil to the cancer cells; and applying an alternating electric field to the cancer cells, the alternating electric field having a frequency between 100 and 400 kHz.

In some instances, at least a portion of the applying is performed simultaneously with at least a portion of the administering.

In some instances, the applying has a duration of at least 72 hours. The application of the electrical field for 72 hours may be accomplished in a single 72 hour interval. Alternatively, the application of the electrical field could be interrupted by breaks. For example, 6 sessions with a duration of 12 hours each, with a 2 hour break between sessions.

In some instances, the frequency of the alternating electric field is between 120 and 180 kHz. In some instances, the frequency of the alternating electric field is between 145 and 155 kHz.

In some instances, the fluorouracil is administered to the cancer cells at a therapeutically effective concentration, and the alternating electric field has a field strength of at least 1 V/cm in at least some of the canccr cells. In some of these instances, the applying has a duration of at least 72 hours and the frequency of the alternating electric field is between 120 and 180 kHz. In some of these instances, the applying has a duration of at least 72 hours and the frequency of the alternating electric field is between 145 and 155 kHz.

In some instances, the administration of the fluorouracil is accomplished by direct administration of the fluorouracil to the cells. In other instances, the fluorouracil can be administered to the cells indirectly.

Another aspect of the invention is directed to a method of reducing viability of gastric cancer cells in a subject. This aspect comprises administering fluorouracil to the gastric cancer cells in the subject; and applying an alternating electric field to the gastric cancer cells in the subject, the alternating electric field having a frequency between 100 and 400 kHz.

In some instances, at least a portion of the applying is performed simultaneously with at least a portion of the administering.

In some instances, the applying has a duration of at least 72 hours. The application of the electrical field for 72 hours may be accomplished in a single 72 hour interval. Alternatively, the application of the electrical field could be interrupted by breaks. For example, 6 sessions with a duration of 12 hours each, with a 2 hour break between sessions.

In some instances, the frequency of the alternating electric field is between 120 and 180 kHz. In some instances, the frequency of the alternating electric field is between 145 and 155 kHz.

In some instances, the fluorouracil is administered to the subject at a therapeutically effective concentration, and the alternating electric field has a field strength of at least 1 V/cm in at least some of the gastric cancer cells of the subject. In some of these instances, the applying has a duration of at least 72 hours and the frequency of the alternating electric field is between 120 and 180 kHz. In some of these instances, the applying has a duration of at least 72 hours and the frequency of the alternating electric field is between 145 and 155 kHz.

In some instances, the administration of the fluorouracil is accomplished by direct administration of the fluorouracil to the subject. In other instances, the fluorouracil can be administered to the subject indirectly.

### EXAMPLES

### Example 1 - Objectives and Design

The objective of one study was to test the efficacy of the combined treatment of FOLFOX (Oxaliplatin, 5-FU and Leucovorin) and TTFields, in gastric cancer cell lines AGS and KATO III. The measured treatment outcome in the study included (1) cytotoxic effect, (2) analysis of apoptosis, (3) clonogenic effect, and (4) overall effect.

Each cell line was seeded in inovitro dishes and treated with each chemotherapy agent (Oxaliplatin, 5-FU and Leucovorin) taken alone, and also with the combination of all three (FOLFOX) in specified concentrations with and without TTFields.

The duration of treatment was 72 Hours at a frequency known to be effective for the two tested cell lines (150kHz) and in intensity of 1.6 V/cm RMS for KATO III and 1.0 V/cm RMS for AGS. During the treatment the media of all dishes was replaced every 24h ("hours").

After 72h of treatment the media and the cells were collected, and cytotoxicity was determined by cell counts. Cells (including cells from the media) were stained for Annexin V and 7AAD for apoptosis analysis. For the testing of the clonogenic effect, cells were seeded for colonies.

After 6-14 days the colonies were fixed, stained and counted to determine clonogenic effect. The overall effect was calculated by multiplying the cytotoxic effect and the clonogenic effect.

### Example 2 - Treatment

Cells were seeded on inovitro dishes and petri dishes 24h before starting the experiments. Media containing the indicated concentration of each chemotherapy component alone or FOLFOX and control media was added to the cells and the dishes were connected to TTFields (as in SOP-BI-009) or grown in 37°C, 5% CO₂ for 72h. Each treatment included 8 repeats (8 dishes). The media was replaced every 24h. At the end of the experiment the cells were collected, and cytotoxicity was determined by cell counts, small number of cells were seeded for colonies and the cells (including cells from the media) were stained for Annexin V and 7AAD for apoptosis analysis.

### Example 3 - Devices and Materials

TTFields were applied at a frequency known to be effective in treating gastric cancer cells (150 kHz) and in intensity of 1.6 V/cm RMS for KATO III and 1.0 V/cm RMS for AGS. A TTFields generator (Inovitro systems) was used to apply the treatment to the cells. This device was developed to imitate the TTFields application on patients.

### Example 4 - Cell Lines

AGS: ATCC^{®} Number: CRL-1739^{™}
Complete growth medium: ATCC-formulated F-12K Medium with 10% FBS and 1% Pen/Strep
Cell Passages: 3-40
Doubling time: 15 h
KATO III :
   TCC^{®} Number: HTB-103^{™}
   Complete growth medium: ATCC-formulated Iscove's Modified Dulbecco's Medium with 20% FBS and 1% Pen/Strep.
   Cell Passages: 3-40
   Doubling time: 37 h

### Example 5 - Chemotherapy

Oxaliplatin: Sigma Rcf: Y0000271 lot# 005P37 expiry: 31/10/2018. Reconstitute to 5mM in PBS. Store at -20°C
5-FU: Sigma Ref: PHR1227 lot# LRAA9039 expiry: 01/12/2020. Reconstitute to 0.4M in DMSO. Store at 4°C
Leucovorin: Sigma Ref: PHR154 lot# LRAA418 expiry: 01/12/2019. Reconstitute to 10mM in water. Store at -20°C

### Example 6 - Statistical Analysis

Cytotoxic effect: Average and standard deviation of cell number of at least 5 dishes from each treatment was calculated in Excel software. The averages from these repeats was calculated to averages and standard error and is presented as percent from control (no drug treatment) in XY graph of points with error bars using GraphPed software. Significant was determine by 2-way ANOVA followed Multiple comparison using GraphPed software.

Apoptotic effect: Average of cell fraction of each marker staining (AnnexinV and 7AAD) of at least 5 dishes from each treatment was calculated in Excel software. The averages from these repeats was calculated to averages and is presented in stacked bar graph using GraphPed software.

Clonogenic effect: The colonies from each well were counted using ImageJ software. Average and standard deviation of colony number of at least 5 wells from each treatment were calculated in excel software. The averages from these repeats was calculated to averages and standard errors and is presented as percent from control (no drug treatment) in XY graph of points with error bars using GraphPed software. Significant was determine by 2-way ANOVA followed Multiple comparison using GraphPed software.

Overall effect: Each colony number was multiply in the percent of cytotoxic effect of its dish and averages and standard deviations of each treatment were calculated in Excel software. The averages from these repeats were calculated to averages and standard errors and are presented as percent from control (no drug treatment) in XY graph of points with error bars using GraphPed software. Significant was determine by 2-way ANOVA followed Multiple comparison using GraphPed software.

The experiments were performed to determine if the elevation in the overall effect of TTFields + chemotherapy group will be significant compared to effect of the equivalent chemotherapy concentration alone or TTFields alone.

### Example 7 - Experimental Set Up and Results

### A. AGS + Oxaliplatin:

Dishes were seeded with 15^{∗}103 cells each 24h before starting the experiments. Media with oxaliplatin was used in concentrations of 25, 50, 100, 200 and 400 nM added to the cells. Each concentration in 8 dishes with TTFields and 8 dishes without TTFields. The TTFields dishes were connected to TTFields for 72h and the dishes without TTFields were grown in normal conditions (37° 5% CO2 Incubator). Media was replaced every 24h.

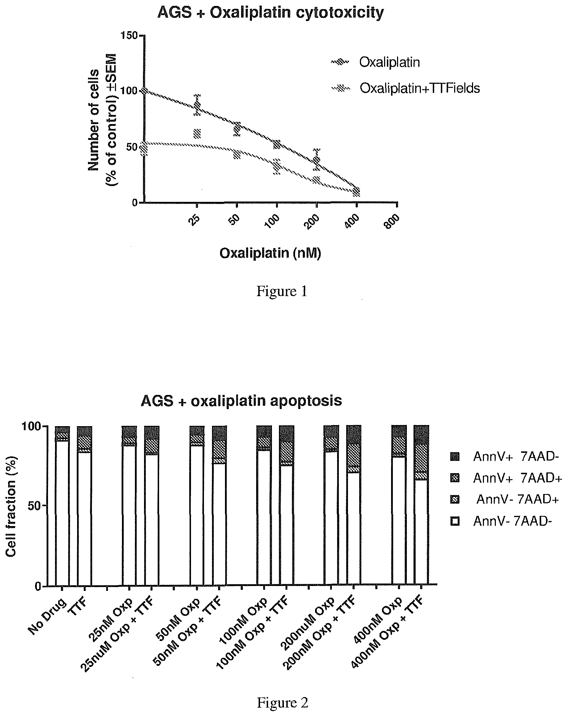

After 72h treatment the media and the cells were collected, the number of cells was counted (as in SOP-BI-009) to establish cytotoxicity. The results in Table 1 and Figure 1 represent at least 3 repeats for each concentration:

**Table 1: Mean and SEM of cell count (% from control) for each Oxaliplatin concentration**

| | **Oxaliplatin** | | | **Oxaliplatin+TTFields** | | |
|---|---|---|---|---|---|---|
| **Oxaliplatin (nM)** | **Mean** | **SEM** | **N** | **Mean** | **SEM** | **N** |
| **10** | 100 | 0 | 7 | 49 | 6 | 6 |
| **25** | 88 | 9 | 3 | 62 | 3 | 4 |
| **50** | 66 | 6 | 4 | 43 | 3 | 4 |
| **100** | 52 | 3 | 4 | 33 | 6 | 4 |
| **200** | 39 | 9 | 5 | 21 | 3 | 5 |
| **400** | 11 | 1 | 3 | 8 | 1 | 3 |

The cells were analyzed to determine the apoptotic effect of AGS and oxaliplatin. The results in Table 2 and Figure 2 represent at least 3 repeats of each concentration:

**Table 2: Mean and SEM of cell apoptosis analysis (% of cell fraction of 7AAD and AnnexinV staining) of each Oxaliplatin concentration**

| | **AnnV- 7AAD+** | | **AnnV+ 7AAD+** | | **AnnV- 7AAD-** | | **AnnV+ 7AAD-** | | |
|---|---|---|---|---|---|---|---|---|---|
| | **Mean** | **SEM** | **Mean** | **SEM** | **Mean** | **SEM** | **Mean** | **SEM** | **N** |
| **No Drug** | 1.44 | 0.58 | 3.84 | 0.64 | 91.26 | 0.99 | 3.48 | 0.49 | 5 |
| **TTFields** | 2.10 | 0.47 | 8.12 | 2.47 | 84.12 | 3.14 | 5.70 | 1.14 | 5 |
| **25nM Oxp** | 1.35 | 0.95 | 4.00 | 0.20 | 88.10 | 4.60 | 6.55 | 3.45 | 2 |
| **25nuM Oxp + TTFields** | 0.75 | 0.35 | 9.00 | 3.60 | 82.60 | 4.20 | 7.65 | 0.25 | 2 |
| **50nM Oxp** | 2.08 | 0.69 | 4.83 | 0.45 | 88.00 | 1.77 | 5.08 | 0.95 | 4 |
| **50nM Oxp + TTFields** | 3.10 | 0.91 | 11.65 | 4.55 | 76.90 | 5.50 | 8.40 | 1.50 | 4 |
| **100nM Oxp** | 1.63 | 1.10 | 7.00 | 1.47 | 84.93 | 2.02 | 6.45 | 1.81 | 4 |
| **100nM Oxp + TTFields** | 2.23 | 0.76 | 12.78 | 5.40 | 75.20 | 7.09 | 9.78 | 2.26 | 4 |
| **200nuM Oxp** | 1.46 | 0.59 | 7.64 | 0.92 | 83.92 | 2.23 | 7.00 | 1.41 | 5 |
| **200nM Oxp + TTFields** | 3.78 | 1.68 | 14.54 | 3.06 | 70.58 | 4.38 | 11.10 | 2.20 | 5 |
| **400nM Oxp** | 1.83 | 0.46 | 10.87 | 2.55 | 80.60 | 1.86 | 6.70 | 0.87 | 3 |
| **400nM Oxp + TTFields** | 4.50 | 0.76 | 17.83 | 5.30 | 66.23 | 7.34 | 11.40 | 2.27 | 3 |

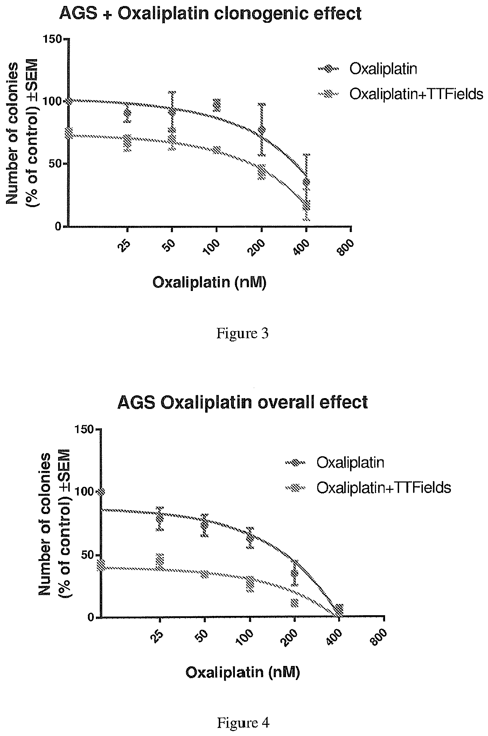

In addition, a small number of cells were seeded for colonies and after 6-14 days the colonies were fixed, stained and counted to determine clonogenic effect. The results in Table 3 and Figure 3 represent at least 2 repeats for each concentration:

**Table 3: Mean and SEM of colonies count (% from control) for each Oxaliplatin concentration**

| | **Oxaliplatin** | | | **Oxaliplatin+TTFields** | | |
|---|---|---|---|---|---|---|
| **Oxaliplatin** (**nM**) | **Mean** | **SEM** | **N** | **Mean** | **SEM** | **N** |
| **10** | 100 | 0 | 5 | 75 | 2 | 6 |
| **25** | 91 | 5 | 2 | 67 | 3 | 3 |
| **50** | 92 | 9 | 3 | 70 | 5 | 3 |
| **100** | 97 | 3 | 2 | 61 | 2 | 3 |
| **200** | 77 | 10 | 4 | 43 | 3 | 4 |
| **400** | 36 | 16 | 2 | 18 | 9 | 2 |

The overall effect was calculated by multiplying the cytotoxic effect and the clonogenic effect. The results in Table 4 and Figure 4 represent at least 2 repeats for each concentration:

**Table 4: Mean and SEM concentration of colonies count multiplying the cytotoxic effect to determine overall effect (% from control) for each Oxaliplatin concentration.**

| | **Oxaliplatin** | | | **Oxaliplatin+TTFields** | | |
|---|---|---|---|---|---|---|
| **Oxaliplatin (nM)** | **Mean** | **SEM** | **N** | **Mean** | **SEM** | **N** |
| 10 | 100 | 0 | 6 | 42 | 4 | 7 |
| 25 | 78 | 9 | 2 | 41 | 5 | 3 |
| 50 | 73 | 8 | 4 | 34 | 2 | 4 |
| 100 | 63 | 8 | 4 | 26 | 6 | 4 |
| 200 | 35 | 10 | 5 | 11 | 2 | 5 |
| 400 | 6 | 3 | 3 | 3 | 1 | 3 |

### Example 8 - AGS + 5-FU

In these experiments, each dish was seeded with 15×10³ cells 24h before starting the experiments.

Media with 5-FU in concentrations of 0.5, 1.5, 4.5, 13.5 and 40 µM was added to the cells in 8 dishes with TTFields, and 8 dishes without TTFields. The TTFields dishes were connected to TTFields for 72h and the dishes without TTFields were grown in normal conditions (37° 5% CO₂ Incubator). Media was replaced every 24h.

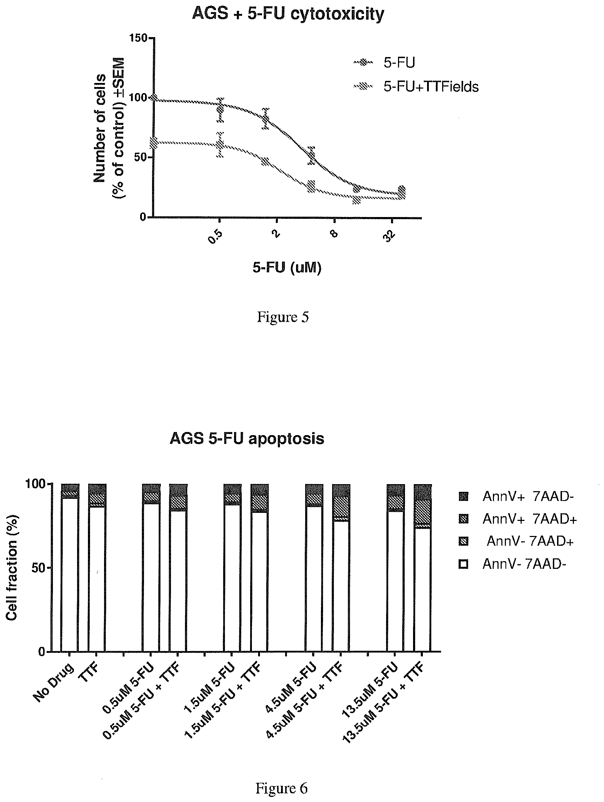

After 72h treatment the media and the cells were collected, the number of cells was counted (to establish cytotoxicity. The results in Table 5 and Figure 5 represent at least 2 repeats of each concentration:

**Table 5: Mean and SEM of at least 2 repeats for each 5-FU concentration of cell count (% from control)**

| | **5-FU** | | | **5-FU+TTFields** | | |
|---|---|---|---|---|---|---|
| **5-FU (uM)** | **Mean** | **SEM** | **N** | **Mean** | **SEM** | **N** |
| **0.1** | 100 | 0 | 6 | 62 | 4 | 6 |
| **0.5** | 90 | 10 | 4 | 61 | 10 | 4 |
| **1.5** | 83 | 8 | 4 | 47 | 2 | 4 |
| **4.5** | 52 | 7 | 4 | 27 | 4 | 4 |
| **13.5** | 24 | 3 | 4 | 15 | 3 | 4 |
| **40** | 25 | 2 | 2 | 19 | 1 | 3 |

The cells were analyzed for apoptosis. The results in Table 6 and Figure 6 represent at least 4 repeats of each concentration:

**Table 6: Mean and SEM of cell apoptosis analysis (percent of cell fraction of 7AAD and AnnexinV staining) for each 5-FU concentration**

| | **AnnV- 7AAD+** | | **AnnV+ 7AAD+** | | **AnnV- 7AAD-** | | **AnnV+ 7AAD-** | | |
|---|---|---|---|---|---|---|---|---|---|
| | **Mean** | **SEM** | **Mean** | **SEM** | **Mean** | **SEM** | **Mean** | **SEM** | **N** |
| **No Drug** | 1.08 | 0.39 | 2.83 | 0.47 | 92.12 | 1.21 | 3.99 | 0.79 | 6 |
| **TTF** | 1.77 | 0.43 | 6.15 | 1.00 | 86.84 | 1.88 | 5.39 | 1.00 | 7 |
| **0.5uM 5-FU** | 1.40 | 0.54 | 5.33 | 1.76 | 88.78 | 1.96 | 4.48 | 0.72 | 4 |
| **0.5uM 5-FU + TTFields** | 1.03 | 0.10 | 8.00 | 1.29 | 84.58 | 2.34 | 6.38 | 1.14 | 4 |
| **1.5uM 5-FU** | 1.23 | 0.63 | 5.48 | 1.18 | 88.25 | 2.37 | 5.03 | 0.85 | 4 |
| **1.5uM 5-FU + TTFields** | 1.28 | 0.19 | 9.05 | 1.75 | 83.80 | 3.24 | 5.90 | 1.43 | 4 |
| **4.5uM 5-FU** | 1.25 | 0.38 | 6.40 | 1.23 | 87.28 | 2.71 | 5.08 | 1.17 | 4 |
| **4.5uM 5-FU + TTFields** | 2.48 | 0.72 | 12.35 | 2.53 | 78.43 | 3.20 | 6.78 | 1.44 | 4 |
| **13.5uM 5-FU** | 0.90 | 0.18 | 8.45 | 0.79 | 84.45 | 2.55 | 6.20 | 1.64 | 4 |
| **13.5uM 5-FU + TTFields** | 2.63 | 0.78 | 14.45 | 4.15 | 74.20 | 6.53 | 8.70 | 2.92 | 4 |

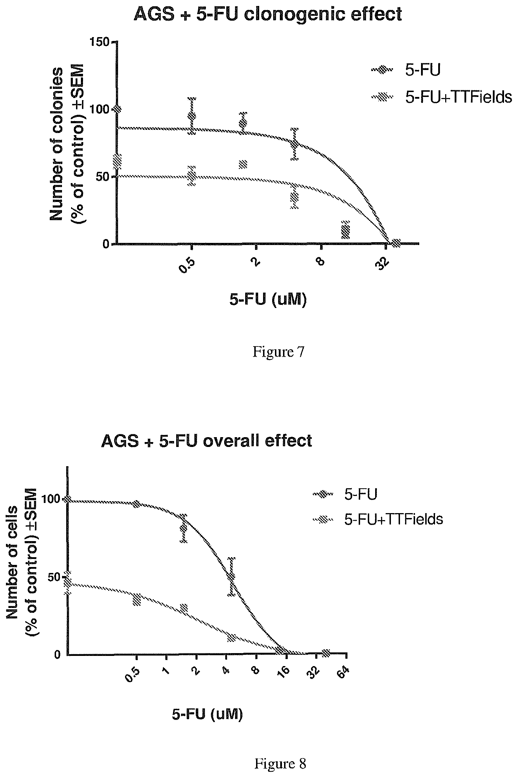

In addition, a small number of cells were seeded for colonies and after 6-14 days the colonies were fixed, stained and counted to determine clonogenic effect. The results in Table 7 and Figure 7 represent at least 2 repeats for each concentration:

**Table 7: Mean and SEM of colonies count (% from control) for each 5-FU concentration**

| | **5-FU** | | | **5-FU+TTFields** | | |
|---|---|---|---|---|---|---|
| **5-FU (uM)** | **Mean** | **SEM** | **N** | **Mean** | **SEM** | **N** |
| **0.1** | 100 | 0 | 5 | 61 | 5 | 5 |
| **0.5** | 95 | 13 | 2 | 51 | 7 | 2 |
| **1.5** | 90 | 8 | 2 | 59 | 0 | 2 |
| **4.5** | 74 | 11 | 3 | 35 | 8 | 2 |
| **13.5** | 9 | 4 | 4 | 11 | 6 | 4 |
| **40** | 0 | 0 | 2 | 0 | 0 | 3 |

The overall effect of 5-FU alone and with TTFields treatment was calculated by multiplying the cytotoxic effect and the clonogenic effect. The results in Table 8 and Figure 8 represent at least 2 repeats for each concentration:

**Table 8: Mean and SEM of colonies count multiplying the cytotoxic effect to determine overall effect (% from control) for each 5-FU concentration**

| | **5-FU** | | | **5-FU+TTFields** | | |
|---|---|---|---|---|---|---|
| **5-FU (uM)** | **Mean** | **SEM** | **N** | **Mean** | **SEM** | **N** |
| **0.1** | 100 | 0 | 6 | 44 | 6 | 7 |
| **0.5** | 97 | 2 | 4 | 36 | 3 | 4 |
| **1.5** | 81 | 9 | 2 | 30 | 1 | 3 |
| **4.5** | 50 | 12 | 4 | 11 | 1 | 3 |
| **13.5** | 2 | 1 | 4 | 2 | 1 | 4 |
| **40** | 0 | 0 | 2 | 0 | 0 | 3 |

### Example 9 - AGS + Leucovorin

Each dish was seeded with 15×10³ cells 24h before starting the experiments.

Media with Leucovorin in concentrations of 0.2, 1, and 5 µM was added to the cells, with each concentration in 8 dishes with TTFields, and 8 dishes without TTFields. The TTFields dishes were connected to TTFields for 72h, and the dishes without TTFields were grown in normal conditions (37° 5% CO₂ Incubator). Media was replaced every 24h.

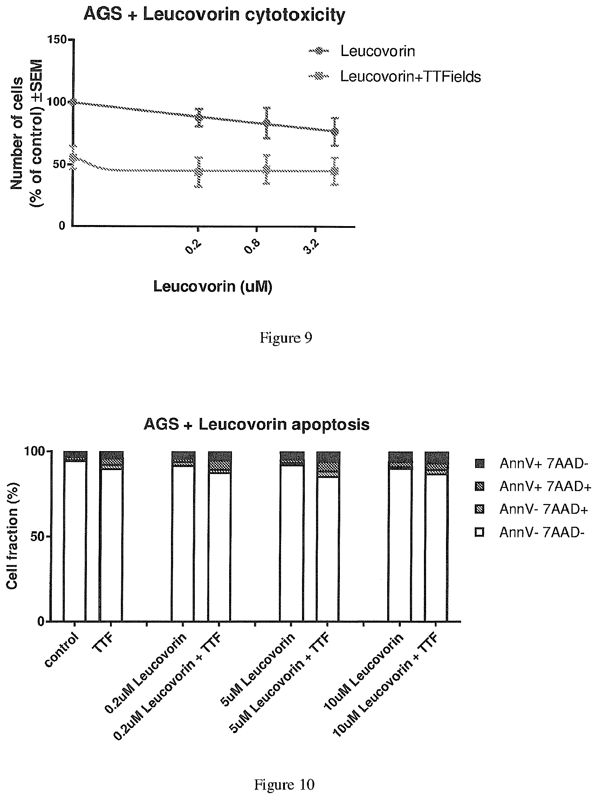

After 72h treatment, the media and the cells were collected, and the number of cells were counted to establish cytotoxicity. The results in Table 9 and Figure 9 represent 2 repeats of each concentration:

**Table 9: Mean and SEM of cell count (% from control) for each Leucovorin concentration**

| | **Leucovorin** | | | **Leucovorin+TTFields** | | |
|---|---|---|---|---|---|---|
| **Leucovorin(uM)** | **Mean** | **SEM** | **N** | **Mean** | **SEM** | **N** |
| **0.001** | 100 | 0 | 2 | 55.5 | 9.5 | 2 |
| **0.2** | 88 | 7 | 2 | 44 | 12 | 2 |
| **1** | 84 | 12 | 2 | 46.5 | 11.5 | 2 |
| **5** | 77 | 11 | 2 | 45 | 11 | 2 |

The cells were analyzed for an apoptotic effect. The results were not repeated because leucovorin alone is not expected to show a large effect on apoptosis. Table 10 and Figure 10 represent the results of one of these experiments.

**Table 10: Results of cell apoptosis analysis (percent of cell fraction of 7AAD and AnnexinV staining) for each Leucovorin concentration**

| | **AnnV-7AAD+** | **AnnV+ 7AAD+** | **AnnV-7AAD-** | **AnnV+ 7AAD-** | **N** |
|---|---|---|---|---|---|
| **control** | 0.66 | 1.85 | 94.66 | 2.83 | 1 |
| **TTFields** | 2.51 | 4.02 | 90 | 3.48 | 1 |
| **0.2uM Leucovorin** | 2.19 | 2.25 | 92.14 | 3.42 | 1 |
| **0.2uM Leucovorin + TTFields** | 2.18 | 5.34 | 87.79 | 4.69 | 1 |
| **5uM Leucovorin** | 1.02 | 2.29 | 92.57 | 4.12 | 1 |
| **5uM Leucovorin + TTFields** | 3.04 | 5.55 | 85.8 | 5.61 | 1 |
| **10uM Leucovorin** | 1.01 | 2.84 | 90.54 | 5.62 | 1 |
| **10uM Leucovorin + TTFields** | 2.72 | 3.74 | 87.27 | 6.27 | 1 |

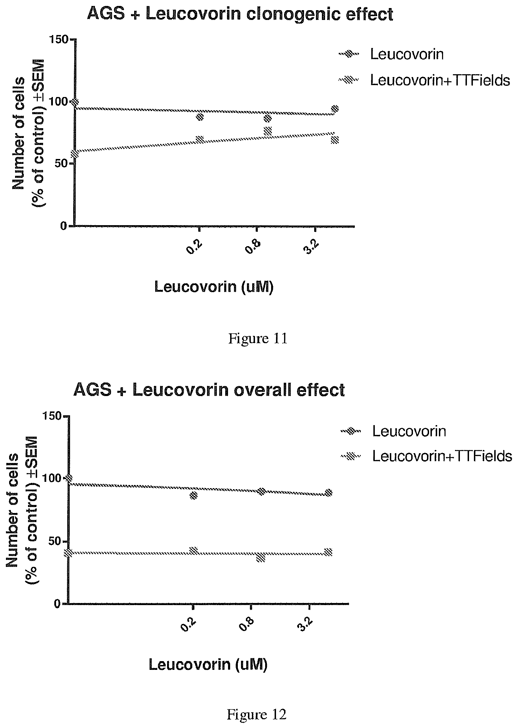

In addition, a small number of cells were seeded for colonies. After 6-14 days ,the colonies were fixed, stained and counted to determine clonogenic effect. The results were not repeated because leucovorin alone is not expected to show a large effect. Table 11 and Figure 11 represent the results of one experiment:

**Table 11: Results of colonies count (% from control) for each Leucovorin concentration**

| | **Leucovorin** | **Leucovorin+TTFields** |
|---|---|---|
| **Leucovorin (uM)** | **Colonies count (% from control)** | **Colonies count (% from control** |
| **0** | 100 | 58 |
| **0.2** | 88 | 70 |
| **1** | 87 | 77 |
| **5** | 95 | 70 |

The overall effect of leucovorin alone with TTFields treatment was calculated by multiplying the cytotoxic effect and the clonogenic effect. Table 12 and Figure 12 represent the results of one experiment:

**Table 12: Results of colonies count multiplying the cytotoxic effect to determine overall effect (% from control)**

| | **Leucovorin** | **Leucovorin+TTFields** |
|---|---|---|
| **Leucovorin (uM)** | **Colonies count (% from control** | **Colonies count (% from control** |
| **0** | 100 | 40.8 |
| **0.2** | 86.6 | 42.9 |
| **1** | 89.7 | 36.7 |
| **5** | 88.8 | 41.7 |

### Example 10 - AGS + FOLFOX

Each dish was seeded with 15×10³ cells each 24h before starting the experiments.

Media with Oxaliplatin alone, 5-FU alone and Leucovorin alone in concentrations that show 15-25% reduction in cell number and a mix of all 3 chemotherapy (FOLFOX) was added to the cells, each chemotherapy in 8 dishes with TTFields, and 8 dishes without TTFields. The concentrations were as follows: oxaliplatin ― 50nM, 5-FU ― 0.5uM, leucovorin ― 5uM.

The TTFields dishes were connected to TTFields for 72h and the dishes without TTFields were grown in normal conditions (37° 5% CO₂ Incubator). The media was replaced every 24h.

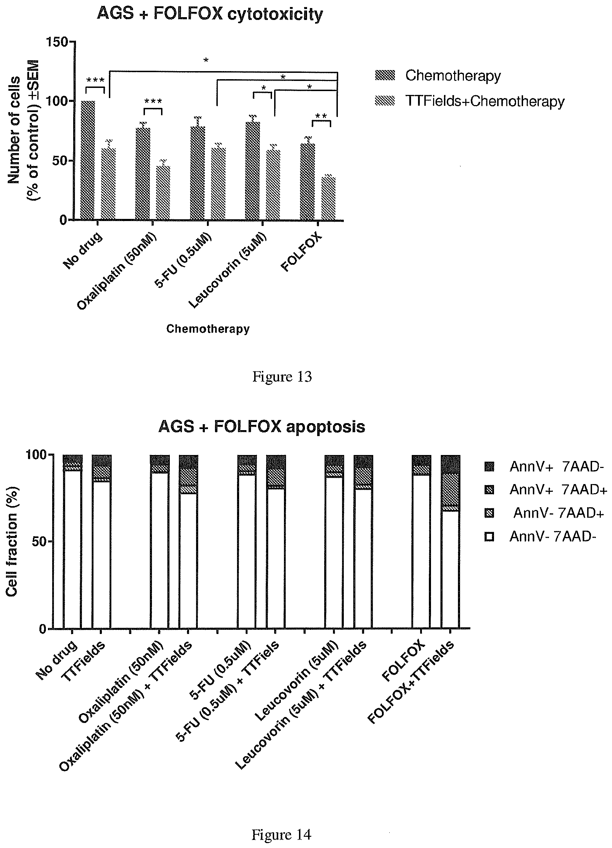

After 72h treatment, the media and the cells were collected, and the number of cells was counted to establish cytotoxicity. The results in Table 13 and Figure 13 represent 3 repeats of each concentration:

**Table 13: Mean and SEM of cell count (% from control) for each Chemotherapy alone and FOLFOX**

| | **Chemotherapy** | | | **TTFields+Chemotherapy** | | |
|---|---|---|---|---|---|---|
| | **Mean** | **SEM** | **N** | **Mean** | **SEM** | **N** |
| **No drug** | 100 | 0 | 3 | 60 | 7 | 3 |
| **Oxaliplatin (50nM)** | 78 | 4 | 3 | 46 | 5 | 3 |
| **5-FU (0.5uM)** | 79 | 8 | 3 | 61 | 4 | 3 |
| **Leucovorin (5uM)** | 83 | 5 | 3 | 59 | 4 | 3 |
| **FOLFOX+TTFields** | 65 | 5 | 3 | 37 | 2 | 3 |

The cells were analyzed for apoptotic effect. The results in Table 14 and Figure 14 represent 2 repeats for each concentration:

**Table 14 Mean and SEM of cell apoptosis analysis (percent of cell fraction of 7AAD and AnnexinV staining) for each chemotherapy alone and FOLFOX**

| | **AnnV-7AAD+** | | **AnnV+ 7AAD+** | | **AnnV-7AAD-** | | **AnnV+ 7AAD-** | | |
|---|---|---|---|---|---|---|---|---|---|
| | **Mean** | **SEM** | **Mean** | **SEM** | **Mean** | **SEM** | **Mean** | **SEM** | **N** |
| **No drug** | 2.2 | 1.8 | 2.6 | 0.6 | 91.4 | 2.2 | 3.8 | 0.2 | 2 |
| **TTFields** | 2.0 | 1.5 | 7.3 | 3.2 | 84.9 | 5.8 | 5.7 | 1.1 | 2 |
| **Oxaliplatin (50nM)** | 0.2 | 0.0 | 4.6 | 1.8 | 90.1 | 2.0 | 5.1 | 0.2 | 2 |
| **Oxaliplatin (50nM) + TTFields** | 4.4 | 4.2 | 10.3 | 5.0 | 78.2 | 9.6 | 7.2 | 0.4 | 2 |
| **5-FU (0.5uM)** | 1.8 | 1.8 | 4.3 | 1.8 | 89.1 | 4.1 | 4.8 | 0.5 | 2 |
| **5-FU (0.5uM) + TTFields** | 1.7 | 0.9 | 10.3 | 4.7 | 80.8 | 6.7 | 7.3 | 1.0 | 2 |
| **Leucovorin (5uM)** | 2.6 | 2.5 | 4.3 | 2.0 | 87.7 | 5.4 | 5.3 | 0.9 | 2 |
| **Leucovorin (5uM) + TTFields** | 2.4 | 2.0 | 10.2 | 3.5 | 80.7 | 5.8 | 6.6 | 0.3 | 2 |
| **FOLFOX** | 0.2 | 0.0 | 5.5 | 2.5 | 89.0 | 2.4 | 5.3 | 0.1 | 2 |
| **FOLFOX + TTFields** | 2.9 | 2.6 | 19.0 | 11.8 | 68.1 | 16.7 | 10.1 | 2.4 | 2 |

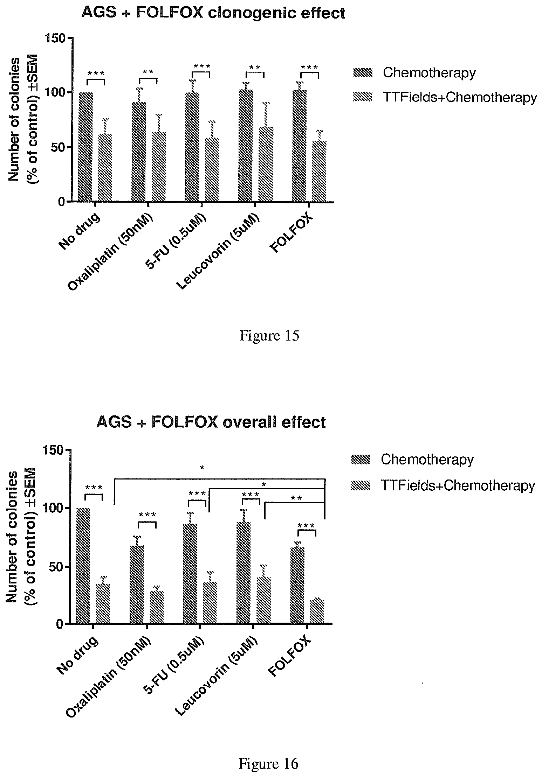

In addition, a small number of cells were seeded for colonies. After 6-14 days, the colonies were fixed, stained, and counted to determine clonogenic effect . The results in Table 15 and figure 15 represent at least 2 repeats for each concentration:

**Table 15: Mean and SEM of colonies count (% from control) for each Chemotherapy alone and FOLFOX**

| | **Chemotherapy** | | | **TTFields+Chemotherapy** | | |
|---|---|---|---|---|---|---|
| | **Mean** | **SEM** | **N** | **Mean** | **SEM** | **N** |
| **No drug** | 100 | 0 | 9 | 62 | 4 | 9 |
| **Oxaliplatin (50nM)** | 91 | 5 | 6 | 64 | 6 | 6 |
| **5-FU (0.5uM)** | 100 | 5 | 5 | 59 | 7 | 5 |
| **Leucovorin (5uM)** | 103 | 3 | 4 | 69 | 11 | 4 |
| **FOLFOX+TTFields** | 103 | 4 | 3 | 56 | 6 | 3 |

The overall effect of the chemotherapy was calculated by multiplying the cytotoxic effect and the clonogenic effect. The results in Table 16 and Figure 16 represent at least 3 repeats for each treatment:

**Table 16: Mean and SEM of colonies count multiplying the cytotoxic effect to determine overall effect (% from control) for each Chemotherapy and FOLFOX**

| | **Chemotherapy** | | | **TTFields+Chemotherapy** | | |
|---|---|---|---|---|---|---|
| | **Mean** | **SEM** | **N** | **Mean** | **SEM** | **N** |
| **No drug** | 100 | 0 | 9 | 35 | 2 | 9 |
| **Oxaliplatin (50nM)** | 68 | 3 | 6 | 29 | 2 | 6 |
| **5-FU (0.5uM)** | 87 | 4 | 5 | 36 | 4 | 5 |
| **Leucovorin (5uM)** | 88 | 5 | 4 | 41 | 5 | 4 |
| **FOLFOX+TTFields** | 66 | 3 | 3 | 21 | 1 | 3 |

### Example 11 - KATO III + Oxaliplatin

Each dish was seeded with 20×10³ cells 24h before starting the experiments.

Media with oxaliplatin was provided in concentrations of 50, 100, 200, 400 and 800 nM and added to the cells, with each concentration in 8 dishes with TTFields and 8 dishes without TTFields. The TTFields dishes were connected to TTFields for 72h and the dishes without TTFields were grown in normal conditions (37° 5% CO₂ Incubator). Media was replaced every 24h.

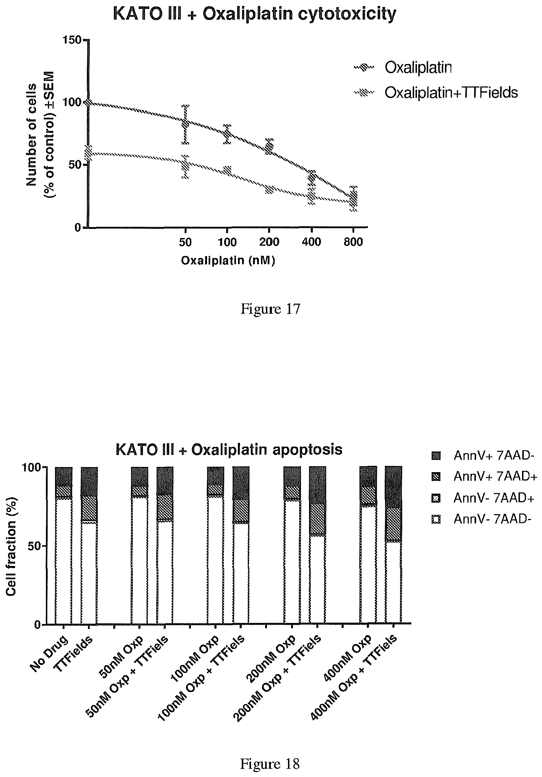

After 72h treatment the media and the cells were collected, the number of cells was counted to establish cytotoxicity. The results in Table 17 and Figure 17 represent at least 2 repeats of each concentration:

**Table 17: Mean and SEM of cell count (% from control) for each Oxaliplatin concentration**

| | **Oxaliplatin (uM)** | | | **Oxaliplatin+TTFields** | | |
|---|---|---|---|---|---|---|
| **Oxaliplatin (nM)** | **Mean** | **SEM** | **N** | **Mean** | **SEM** | **N** |
| **10** | 100 | 0 | 4 | 60 | 5 | 4 |
| **50** | 82 | 15 | 3 | 48 | 9 | 3 |
| **100** | 75 | 7 | 4 | 46 | 3 | 4 |
| **200** | 64 | 5 | 4 | 30 | 2 | 4 |
| **400** | 39 | 5 | 4 | 24 | 6 | 4 |
| **800** | 25 | 8 | 2 | 21 | 8 | 2 |

The cells were analyzed for apoptotic effect. The results in Table 18 and Figure 18 represent at least 3 repeats of each concentration:

**Table 18: Mean and SEM of cell apoptosis analysis (percent of cell fraction of 7AAD and AnnexinV staining) for each Oxaliplatin concentration**

| | **AnnV- 7AAD+** | | **AnnV+ 7AAD+** | | **AnnV- 7AAD-** | | **AnnV+ 7AAD-** | | |
|---|---|---|---|---|---|---|---|---|---|
| | **Mean** | **SEM** | **Mean** | **SEM** | **Mean** | **SEM** | **Mean** | **SEM** | **N** |
| **No Drug** | 1.27 | 1.00 | 6.81 | 1.69 | 80.59 | 2.21 | 11.34 | 4.74 | 3 |
| **TTFields** | 1.76 | 1.33 | 15.84 | 6.54 | 65.14 | 2.03 | 17.26 | 5.84 | 3 |
| **50nM Oxp** | 1.30 | 0.83 | 6.09 | 0.99 | 81.25 | 3.10 | 11.36 | 4.45 | 3 |
| **50nM Oxp + TTFields** | 1.31 | 0.68 | 16.12 | 2.59 | 66.01 | 0.58 | 16.57 | 2.68 | 3 |
| **100nM Oxp** | 1.32 | 0.83 | 6.42 | 0.75 | 81.71 | 2.07 | 10.55 | 3.24 | 3 |
| **100nM Oxp + TTFields** | 0.72 | 0.18 | 14.83 | 5.13 | 64.63 | 7.47 | 19.81 | 2.23 | 3 |
| **200nM Oxp** | 1.01 | 0.54 | 8.42 | 0.94 | 79.10 | 1.91 | 11.47 | 3.29 | 3 |
| **200nM Oxp + TTFields** | 0.86 | 0.45 | 19.97 | 2.91 | 56.57 | 5.07 | 22.60 | 2.15 | 3 |
| **400nM Oxp** | 1.42 | 1.06 | 11.24 | 0.68 | 75.23 | 0.81 | 12.11 | 2.33 | 3 |
| **400nM Oxp + TTFields** | 0.65 | 0.23 | 21.45 | 1.86 | 52.54 | 5.26 | 25.36 | 4.30 | 3 |

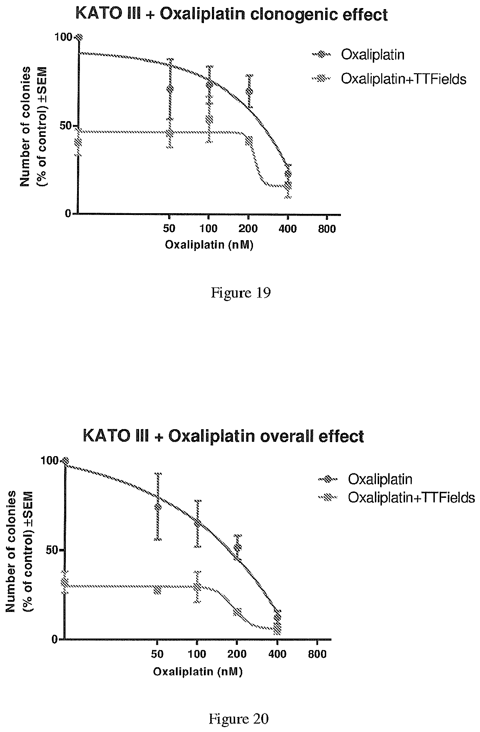

In addition, a small number of cells were seeded for colonies, and after 6-14 days the colonies were fixed, stained, and counted to determine clonogenic effect. The results in Table 19 and Figure 19 represent 2 repeats of each concentration:

**Table 19: Mean and SEM of colonies count (% from control) for each Oxaliplatin concentration**

| | **Oxaliplatin (uM)** | | | **Oxaliplatin+TTFields** | | |
|---|---|---|---|---|---|---|
| **Oxaliplatin (nM)** | **Mean** | **SEM** | **N** | **Mean** | **SEM** | **N** |
| 10 | 100 | 0 | 2 | 41 | 8 | 2 |
| 50 | 71 | 17 | 2 | 46 | 8 | 2 |
| 100 | 74 | 11 | 2 | 54 | 13 | 2 |
| 200 | 70 | 9 | 2 | 42 | 2 | 2 |
| 400 | 23 | 5 | 2 | 17 | 7 | 2 |

The overall effect was calculated by multiplying the cytotoxic effect and the clonogenic effect. The results in Table 20 and Figure 20 represent 2 repeats of each concentration:

**Table 20: Mean and SEM of colonies count multiplying the cytotoxic effect to determine overall effect (% from control) for each Oxaliplatin concentration**

| | **Oxaliplatin (uM)** | | | **Oxaliplatin+TTFields** | | |
|---|---|---|---|---|---|---|
| **Oxaliplatin (nM)** | **Mean** | **SEM** | **N** | **Mean** | **SEM** | **N** |
| 10 | 100 | 0 | 2 | 32 | 6 | 2 |
| 50 | 75 | 19 | 2 | 28 | 2 | 2 |
| 100 | 65 | 13 | 2 | 30 | 9 | 2 |
| 200 | 52 | 7 | 2 | 16 | 1 | 2 |
| 400 | 13 | 4 | 2 | 6 | 3 | 2 |

### Example 12 - KATO III + 5-FU

Each dish was seeded with 20×10³ cells 24h before starting the experiments.

Media with 5-FU in concentrations of 1.5, 4.5, 13.5 and 40 µM was added to the cells, with each concentration in 8 dishes with TTFields, and 8 dishes without TTFields. The TTFields dishes were connected to TTFields for 72h, and the dishes without TTFields were grown in normal conditions (37° 5% CO₂ Incubator). Media was replaced every 24h.

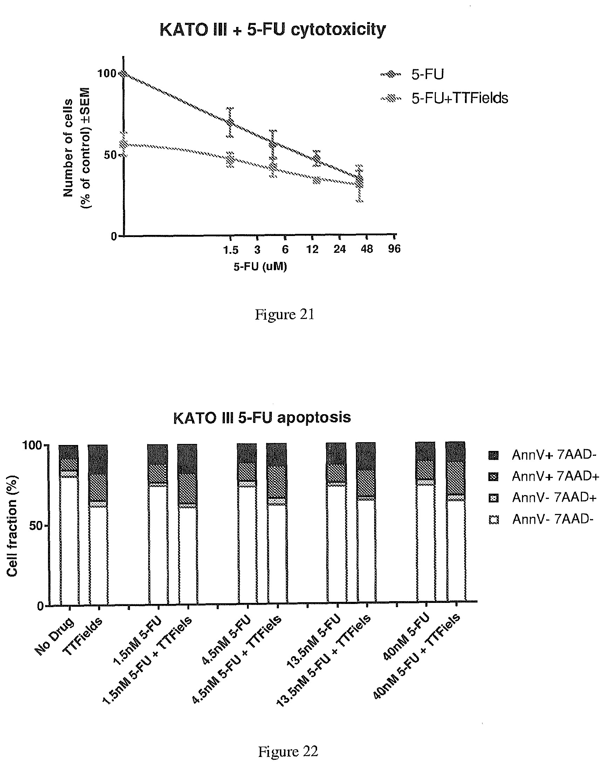

After 72h treatment, the media and the cells were collected, the number of cells was counted to establish cytotoxicity. The results in Table 21 and Figure 21 represent at least 3 repeats of each concentration:

**Table 21: Mean and SEM of cell count (% from control) for each 5-FU concentration**

| | **5-FU** | | | **5-FU+TTFields** | | |
|---|---|---|---|---|---|---|
| **5-FU (uM)** | **Mean** | **SEM** | **N** | **Mean** | **SEM** | **N** |
| **0.1** | 100 | 0 | 4 | 57 | 7 | 4 |
| **1.5** | 70 | 9 | 4 | 47 | 4 | 4 |
| **4.5** | 56 | 9 | 4 | 42 | 6 | 4 |
| **13.5** | 47 | 4 | 4 | 34 | 2 | 4 |
| **40** | 34 | 5 | 3 | 32 | 11 | 4 |

The cells were analyzed for apoptotic effect. The results in Table 22 and Figure 22 represent at least 3 repeats of each concentration:

**Table 22: Mean and SEM of cell apoptosis analysis (percent of cell fraction of 7AAD and Annexin V staining) for each 5-FU concentration**

| | **AnnV- 7AAD+** | | **AnnV+ 7AAD+** | | **AnnV- 7AAD-** | | **AnnV+ 7AAD-** | | |
|---|---|---|---|---|---|---|---|---|---|
| | **Mean** | **SEM** | **Mean** | **SEM** | **Mean** | **SEM** | **Mean** | **SEM** | **N** |
| **No Drug** | 4.04 | 2.11 | 7.44 | 0.91 | 80.77 | 3.57 | 7.75 | 1.67 | 4 |
| **TTFields** | 3.62 | 1.00 | 17.24 | 1.81 | 61.79 | 6.65 | 17.36 | 5.31 | 4 |
| **1.5nM 5-FU** | 1.95 | 1.20 | 11.98 | 2.19 | 74.64 | 7.00 | 11.44 | 3.75 | 4 |
| **1.5nM 5-FU + TTFields** | 2.24 | 0.74 | 19.12 | 2.63 | 61.16 | 7.31 | 17.49 | 5.94 | 4 |
| **4.5nM 5-FU** | 3.59 | 1.27 | 11.92 | 1.87 | 73.50 | 7.07 | 11.00 | 4.53 | 4 |
| **4.5nM 5-FU + TTFields** | 4.25 | 2.09 | 20.44 | 3.90 | 61.98 | 8.52 | 13.34 | 3.65 | 4 |
| **13.5nM 5-FU** | 2.22 | 0.99 | 11.72 | 1.94 | 73.64 | 6.78 | 12.44 | 4.69 | 4 |
| **13.5nM 5-FU + TTFields** | 2.01 | 0.41 | 17.18 | 2.61 | 64.61 | 7.56 | 16.21 | 6.05 | 4 |
| **40nM 5-FU** | 3.42 | 0.59 | 12.48 | 1.00 | 73.55 | 2.49 | 10.55 | 1.39 | 3 |
| **40nM 5-FU + TTFields** | 3.58 | 0.89 | 21.25 | 4.43 | 63.76 | 4.92 | 11.40 | 1.72 | 3 |

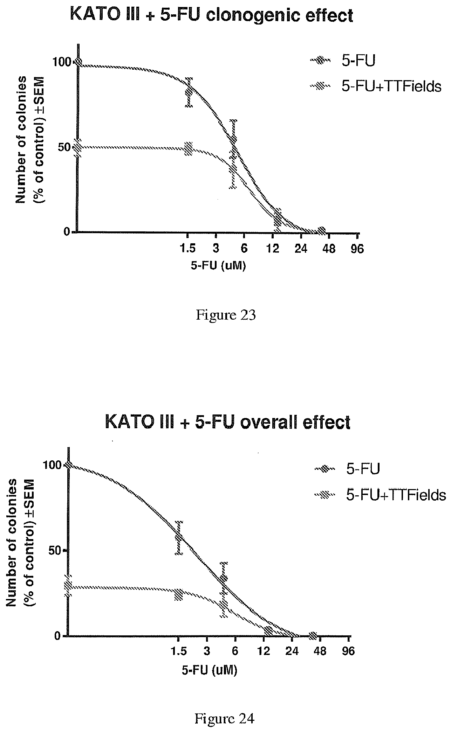

In addition, a small number of cells were seeded for colonies, and after 6-14 days the colonies were fixed, stained, and counted to determine clonogenic effect. The results in Table 23 and Figure 23 represent at least 3 repeats of each concentration:

**Table 23: Mean and SEM of colonies count (% from control) for each 5-FU concentration**

| | **5-FU** | | | **5-FU+TTFields** | | |
|---|---|---|---|---|---|---|
| **5-FU (uM)** | **Mean** | **SEM** | **N** | **Mean** | **SEM** | **N** |
| **0.1** | 100 | 0 | 4 | 50 | 5 | 4 |
| **1.5** | 83 | 8 | 4 | 50 | 3 | 4 |
| **4.5** | 55 | 11 | 4 | 38 | 11 | 4 |
| **13.5** | 9 | 4 | 4 | 8 | 6 | 4 |
| **40** | 2 | 1 | 3 | 1 | 0 | 3 |

The overall effect was calculated by multiplying the cytotoxic effect and the clonogenic effect. The results in Table 24 and Figure 24 represent at least 3 repeats of each concentration:

**Table 24: Mean and SEM of colonies count multiplying the cytotoxic effect to determine overall effect (% from control) for each 5-FU concentration**

| | **5-FU** | | | **5-FU+TTFields** | | |
|---|---|---|---|---|---|---|
| **5-FU (uM)** | **Mean** | **SEM** | **N** | **Mean** | **SEM** | **N** |
| **0.1** | 100 | 0 | 4 | 30 | 6 | 4 |
| **1.5** | 58 | 9 | 4 | 24 | 3 | 4 |
| **4.5** | 34 | 9 | 4 | 18 | 7 | 4 |
| **13.5** | 4 | 2 | 4 | 3 | 2 | 4 |
| **40** | 0 | 0 | 3 | 0 | 0 | 3 |

### Example 13 - KATO III + Leucovorin

Dishes were seeded with 20×10³ cells each 24h before starting the experiments.

Media with leucovorin in concentrations of 2.5, 5 and 10 µM was added to the cells, with each concentration in 8 dishes with TTFields, and 8 dishes without TTFields. The TTFields dishes were connected to TTFields for 72h, and the dishes without TTFields were grown in normal conditions (37° 5% CO2 Incubator). Media was replaced every 24h.

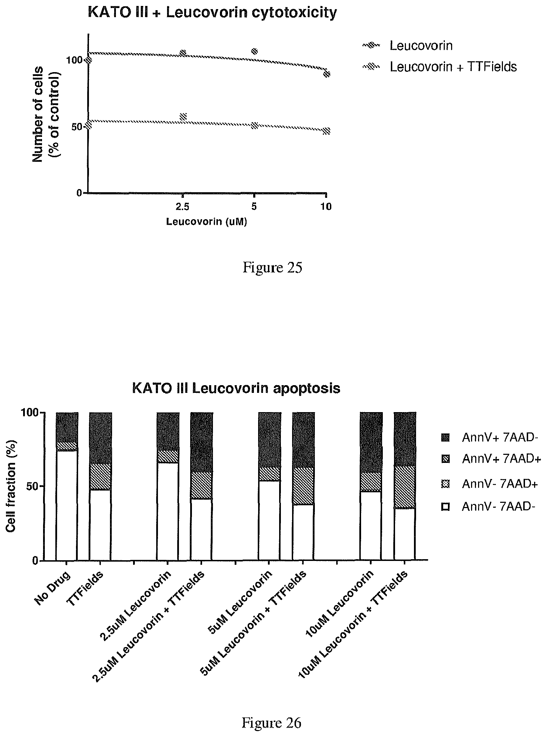

After 72h treatment the media and the cells were collected, the number of cells was counted to establish cytotoxicity. The experiment was not repeated because leucovorin alone is not expected to show a large cytotoxic effect. Table 25 and Figure 25 represent the results of one experiment:

**Table 25: Results of cells count (% from control) for each Leucovorin concentration**

| | **Leucovorin** | **Leucovorin + TTFields** |
|---|---|---|
| **Leucovorin (uM)** | **cell count (% from control)** | **cell count (% from control)** |
| 1 | 100 | 51 |
| 2.5 | 106 | 58 |
| 5 | 107 | 51 |
| 10 | 90 | 47 |

The cells were analyzed for apoptotic effect. The results were not repeated because leucovorin alone is not expected to show a large effect on apoptosis. Table 26 and Figure 26 represent the results of one experiment:

**Table 26: Results of cell apoptosis analysis (percent of cell fraction of 7AAD and AnnexinV staining) for each Leucovorin concentration**

| | **AnnV-7AAD+** | **AnnV+ 7AAD+** | **AnnV-7AAD-** | **AnnV+ 7AAD-** | **N** |
|---|---|---|---|---|---|
| **No Drug** | 0.31 | 6 | 74.66 | 19.03 | 1 |
| **TTFields** | 0.24 | 17.8 | 48.1 | 33.85 | 1 |
| **2.5uM Leucovorin** | 0.06 | 8.51 | 66.57 | 24.86 | 1 |
| **2.5uM Leucovorin + TTFields** | 0.3 | 18.45 | 41.82 | 39.43 | 1 |
| **5uM Leucovorin** | 0.12 | 9.4 | 53.98 | 36.5 | 1 |
| **5uM Leucovorin + TTFields** | 0.2 | 25.55 | 37.69 | 36.56 | 1 |
| **10uM Leucovorin** | 1.01 | 12.64 | 46.5 | 39.85 | 1 |
| **10uM Leucovorin + TTFields** | 0.4 | 28.48 | 35.3 | 35.82 | 1 |

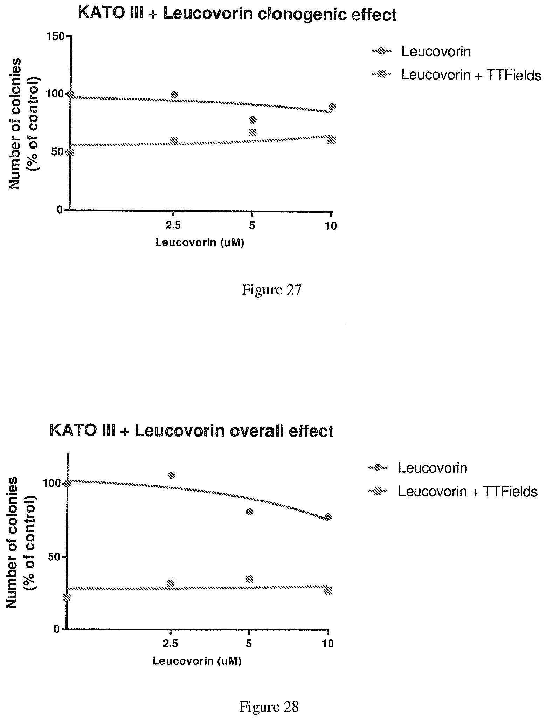

In addition, a small number of cells were seeded for colonies, and after 6-14 days the colonies were fixed, stained, and counted to determine clonogenic effect. The results were not repeated because leucovorin alone is not expected to show a large effect. Table 27 and Figure 27 represent the results of one experiment:

**Table 27: Results of colonies count (% from control) for each Leucovorin concentration**

| | **Leucovorin** | **Leucovorin + TTFields** |
|---|---|---|
| **Leucovorin (uM)** | **colonies count (% from control)** | **colonies count (% from control)** |
| **1** | 100 | 50 |
| **2.5** | 100 | 60 |
| **5** | 79 | 68 |
| **10** | 91 | 62 |

The overall effect was calculated by multiplying the cytotoxic effect and the clonogenic effect. Table 28 and Figure 28 represent the results of one experiment:

**Table 28: Results of colonies count multiplying the cytotoxic effect to determine overall effect (% from control)**

| | **Leucovorin** | **Leucovorin + TTFields** |
|---|---|---|
| **Leucovorin (uM)** | **colonies count (% from control)** | **colonies count (% from control)** |
| **1** | 100 | 22 |
| **2.5** | 106 | 32 |
| **5** | 81 | 35 |
| **10** | 78 | 27 |

### Example 14 - KATO III + FOLFOX

Dishes were seeded with 20X103 cells each 24h before starting the experiments.

Media with oxaliplatin alone, 5-FU alone and leucovorin alone in concentrations that show 15-25% reduction in cell number, and a mix of all 3 chemotherapies (FOLFOX) was added to the cells, with each chemotherapy in 8 dishes with TTFields, and 8 dishes without TTFields. The exact concentrations were as follow: oxaliplatin ― 50nM; 5-FU ― 1.5uM; and Leucovorin ― 5uM.

The TTFields dishes were connected to TTFields for 72h, and the dishes without TTFields were grown in normal conditions (37° 5% CO2 Incubator). The media was replaced every 24h.

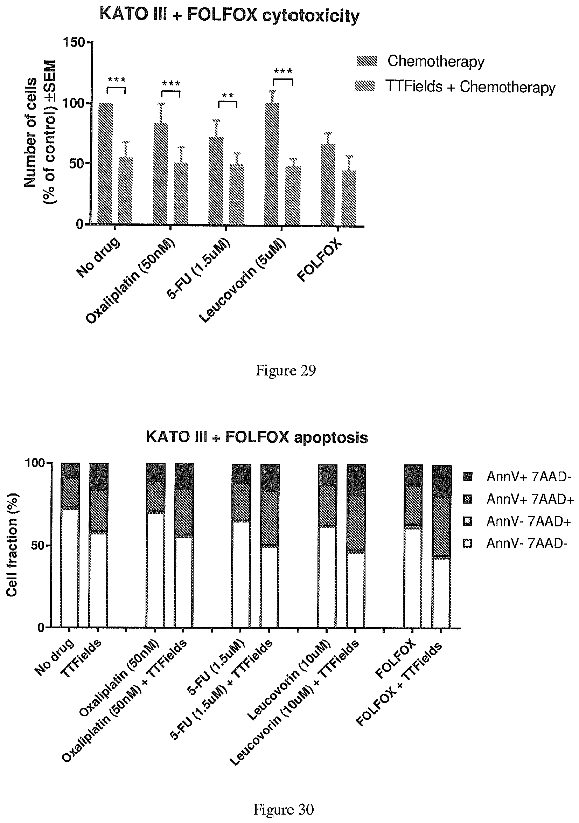

After 72h treatment the media and the cells were collected, the number of cells was counted to establish cytotoxicity. The results in Table 29 and Figure 29 represent at least 3 repeats of each concentration:

**Table 29: Mean and SEM of cell count (% from control) for each Chemotherapy alone and FOLFOX**

| | **Chemotherapy** | | | **TTFields+Chemotherapy** | | |
|---|---|---|---|---|---|---|
| | **Mean** | **SEM** | **N** | **Mean** | **SEM** | **N** |
| **No drug** | 100 | 0 | 11 | 55 | 4 | 11 |
| **Oxaliplatin (50nM)** | 84 | 7 | 6 | 51 | 5 | 6 |
| **5-FU (1.5uM)** | 73 | 5 | 7 | 50 | 4 | 7 |
| **Leucovorin (5uM)** | 102 | 5 | 4 | 49 | 3 | 4 |
| **FOLFOX** | 67 | 5 | 3 | 46 | 6 | 4 |

The cells were analyzed for apoptotic effect. The results in Table 30 and Figure 30 represent 2 repeats of each concentration:

**Table 30: Mean and SEM of cell apoptosis analysis (percent of cell fraction of 7AAD and AnnexinV staining) for each chemotherapy alone and FOLFOX**

| | **AnnV- 7AAD+** | | **AnnV+ 7AAD+** | | **AnnV- 7AAD-** | | **AnnV+ 7AAD-** | | |
|---|---|---|---|---|---|---|---|---|---|
| | **Mean** | **SEM** | **Mean** | **SEM** | **Mean** | **SEM** | **Mean** | **SEM** | **N** |
| **No drug** | 1.40 | 1.18 | 17.42 | 10.30 | 72.62 | 12.46 | 8.57 | 0.98 | 2 |
| **TTF** | 1.43 | 1.11 | 24.75 | 13.35 | 57.96 | 11.68 | 15.86 | 2.78 | 2 |
| **Oxaliplatin (50nM)** | 1.12 | 0.77 | 18.07 | 7.64 | 70.54 | 11.43 | 10.28 | 3.02 | 2 |
| **Oxaliplatin (50nM) + TTFields** | 1.32 | 0.74 | 27.60 | 9.16 | 55.94 | 9.61 | 15.15 | 0.29 | 2 |
| **5-FU (1.5uM)** | 0.96 | 0.61 | 22.22 | 12.30 | 65.47 | 14.46 | 11.36 | 1.56 | 2 |
| **5-FU (1.5uM) + TTFields** | 1.39 | 0.84 | 32.59 | 16.71 | 49.94 | 14.59 | 16.09 | 2.96 | 2 |
| **Leucovorin (10uM)** | 0.80 | 0.53 | 24.39 | 16.29 | 62.46 | 17.73 | 12.36 | 0.92 | 2 |
| **Leucovorin (10uM) + TTFields** | 1.32 | 0.74 | 33.83 | 16.89 | 46.62 | 14.74 | 18.24 | 2.88 | 2 |
| **FOLFOX** | 2.03 | 0.01 | 23.32 | 10.82 | 61.91 | 12.76 | 12.75 | 1.93 | 2 |
| **FOLFOX + TTFields** | 1.43 | 0.91 | 36.29 | 16.66 | 43.36 | 16.11 | 18.93 | 1.47 | 2 |

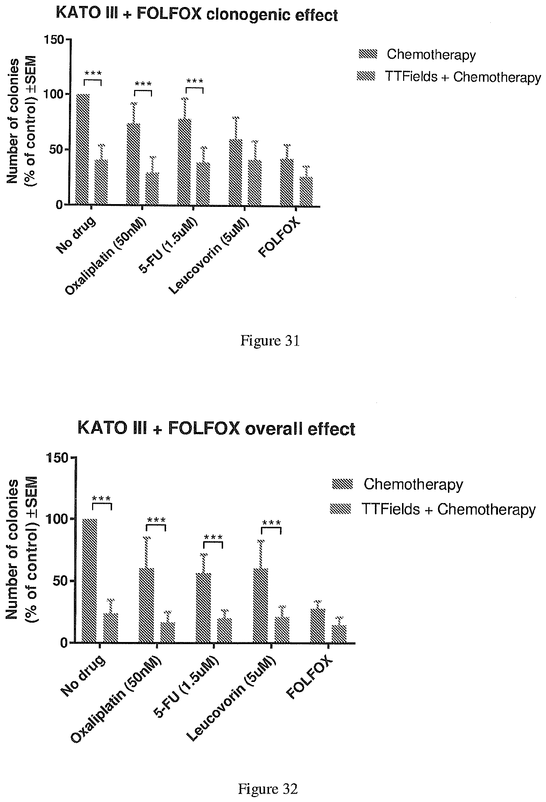

In addition, a small number of cells were seeded for colonies, and after 6-14 days the colonies were fixed, stained and counted to determine clonogenic effect. The results in Table 31 and Figure 31 represent at least 4 repeats of each treatment:

**Table 31: Mean and SEM of colonies count (% from control) for each Chemotherapy alone and FOLFOX**

| | **Chemotherapy** | | | **TTFields+Chemotherapy** | | |
|---|---|---|---|---|---|---|
| | **Mean** | **SEM** | **N** | **Mean** | **SEM** | **N** |
| **No drug** | 100 | 0 | 11 | 41 | 4 | 11 |
| **Oxaliplatin (50nM)** | 67 | 10 | 6 | 30 | 6 | 6 |
| **5-FU (1.5uM)** | 79 | 6 | 8 | 39 | 5 | 8 |
| **Leucovorin (5uM)** | 61 | 9 | 5 | 42 | 7 | 5 |
| **FOLFOX+TTFields** | 43 | 6 | 4 | 27 | 4 | 6 |

The overall effect was calculated by multiplying the cytotoxic effect and the clonogenic effect. The results in Table 32 and Figure 32 represent at least 4 repeats of each treatment:

**Table 32: Mean and SEM of colonies count multiplying the cytotoxic effect to determine overall effect (% from control) for each chemotherapy and FOLFOX**

| | **Chemotherapy** | | | **TTFields + Chemotherapy** | | |
|---|---|---|---|---|---|---|
| | **Mean** | **SEM** | **N** | **Mean** | **SEM** | **N** |
| **No drug** | 100 | 0 | 11 | 24 | 3 | 11 |
| **Oxaliplatin (50nM)** | 61 | 10 | 6 | 17 | 4 | 6 |
| **5-FU (1.5uM)** | 57 | 5 | 8 | 20 | 2 | 8 |
| **Leucovorin (5uM)** | 61 | 10 | 5 | 21 | 4 | 5 |
| **FOLFOX+TTFields** | 28 | 3 | 4 | 14 | 3 | 6 |

### Example 15 - Acceptance Criteria

(a) All the experiments met the following acceptance criteria:
(b) Each repeat contains at least 5 dishes meeting the criteria specified in section 6iii and 6iv
(c) Each experiment was repeated at least 2 times.
(d) Dishes were disqualified if the log documentation shows outlier measurements (temperature <35.0°C or >39.0°C; resistance < 50 Ohm or > 400 Ohm) indicating a technical failure.
(e) Dishes were disqualified even if there was no indication for technical failure if the cytotoxic effect measured in a specific dish was < (average of other dishes-(2×standard deviation of the other dishes)) or > (average of other dishes+ (2×standard deviation of the other dishes)).

### Results for AGS + Oxaliplatin

The cytotoxic effect of Oxaliplatin + TTFields compared to the effect of Oxaliplatin alone is significantly higher in AGS cells (Two-way ANOVA P<0.001). In the apoptosis assay, we can see elevation in the percent of the remaining cells that undergo apoptosis in different stages. This elevation is higher in Oxaliplatin + TTFields treated cells compared to Oxaliplatin alone treated cells. This elevation in apoptosis is translated to clonogenic effect and overall effect and the results show significant reduction in colonies number of Oxaliplatin + TTFields treated cells compared to Oxaliplatin alone treated cells (Two-way ANOVA P<0.001).

### Results for AGS+ 5-FU

The cytotoxic effect of 5-FU + TTFields compared to 5-FU alone effect is significantly higher in AGS cells (Two-way ANOVA P<0.001). In the apoptosis assay, we can see elevation in the percent of the remaining cells that undergo apoptosis in different stages. This elevation is higher in 5-FU + TTFields treated cells compared to 5-FU alone treated cells. This elevation in apoptosis is translated to clonogenic effect and overall effect and the results show significant reduction in colonies number in 5-FU + TTFields treated cells compared to 5-FU alone treated cells (Two-way ANOVA P<0.001). Notably, the interaction between Fluorouracil and TTFields is synergistic based on the overall effect as shown in AGS cells.

### Results for AGS + Leucovorin

As expected, Leucovorin alone docs not show cytotoxic, apoptotic and clonogenic effect on AGS cells. The only observe effect was due to TTFields.

### Results for AGS + FOLFOX

The cytotoxic effect of FOLFOX + TTFields compared to FOLFOX alone effect is significantly higher (Two-way ANOVA followed Multiple comparison P<0.001 (except from 5-FU)). In addition, the cytotoxic effect of TTFields + FOLFOX compared to TTFields effect alone is also significantly higher. In the apoptosis assay, we can see elevation in the percent of the remaining cells that undergo apoptosis in different stages. This elevation is clearly higher in FOLFOX + TTFields treated cells compared to FOLFOX alone treated cells. In the colony assay, the effect of TTFields in each chemotherapy is significant but there is no additional effect of the chemotherapy. The overall effect of each chemotherapy and FOLFOX + TTFields compared to each chemotherapy alone, FOLFOX alone and TTFields alone is significantly higher (Two-way ANOVA followed Multiple comparison P<0.05).

### Results for KATO III + Oxaliplatin

The cytotoxic effect of Oxaliplatin + TTFields compared to Oxaliplatin effect alone is significantly higher in KATO III cells (Two-way ANOVA P<0.001). In the apoptosis assay, we can see elevation in the percent of the remaining cells that undergo apoptosis in different stages. This elevation is higher in Oxaliplatin + TTFields treated cells compared to Oxaliplatin alone treated cells. This elevation in apoptosis is translated to clonogenic effect and overall effect and the results show significant reduction in colonies number in Oxaliplatin + TTFields treated cells compared to Oxaliplatin alone treated cells (Two-way ANOVA P<0.001). The clonogenic and overall effects of TTFields alone in KATO III is very high (40% and 32% respectively). Therefore, there is no additional effect in the low Oxaliplatin concentration.

### Results for KATO III + 5-FU

The cytotoxic effect of 5-FU + TTFields compared to 5-FU effect alone is significantly higher in KATO III cells (Two-way ANOVA P<0.001). In the apoptosis assay, we can see elevation in the percent of the remaining cells that undergo apoptosis in different stages. This elevation is higher in 5-FU + TTFields treated cells compared to 5-FU alone treated cells. This elevation in apoptosis is translated to clonogenic effect and overall effect and the results show significant reduction in colonies number of 5-FU + TTFields treated cells compared to 5-FU alone treated cells (Two-way ANOVA P<0.001).

### Results for KATO III + Leucovorin

As expected, Leucovorin alone does not show cytotoxic, apoptotic and clonogenic effect on KATO III cells. The only observed effect was due to TTFields.

### Results for KATO III + FOLFOX

The cytotoxic effect of FOLFOX + TTFields compared to FOLFOX alone is higher (but not significant), similar to the effect of FOLFOX + TTFields compared to TTFields alone. This results are well reflected in the apoptosis assay and in clonogenic and overall effect. A possible explanation is that TTFields alone and FOLFOX alone show high cytotoxic and clonogenic effect on KATO III cells so the combination is not very effective, though not antagonist.

### General Conclusion

According to the results for the AGS cells there is significant elevation in overall effect in each chemotherapy alone and in FOLFOX in combination with TTFields. The results for the KATO III cells show the same trend but were not significant, maybe due to this cell line high sensitivity to each treatment alone.

Note that while these experiments were performed using the frequencies, field intensities, and durations specified in Appendix A, those parameters may be varied. For example, the frequency could be between 145 and 155 kHz, between 120 and 180 kHz, or between 100 and 400 kHz; the electric field intensity could be between 0.5 and 5 V/cm, or at least 1 V/cm; and the duration could be anything longer than 8 hours.

### Example 16 -Study Currently Underway Regarding Treatment of Gastric Cancer with TTFields combined with Xelox or the Individual Components Thereof

Study device ― TTFields - Model: NovoTTF-100L(P) 150KHz
Treatment time: Average (monthly average) use of at least 18 hours per day
Study drugs: oxaliplatin: 50 mg/vial; capecitabine: 500 mg/tablet, 12 tablets/box
XELOX Chemotherapy: Oxaliplatin: 130mg/m2, iv GTT (Glucose Tolerance Test) for 2-6 hours on day1 of each treatment cycle, 3 weeks (21 days) per treatment cycle; Capecitabine: 1000mg/m2, PO, BID on day1-14 of each treatment cycle, 3 weeks (21 days) per treatment cycle;
XELOX: 21 days per treatment cycle.

Investigator will prescribe patients with oxaliplatin, capecitabine, accepted brand-name drugs or generic drugs for corresponding indications that have been approved by NMPA in China according to the local clinical practice. The dosage should follow the therapeutic dose of the protocol.

For HER-2 positive patients, trastuzumab is allowed based on XELOX regimen, which is as below.

Oxaliplatin: 130 mg/m2, iv gtt for 2-6 hours on day 1, once every 3 weeks;

Capecitabine: 1000 mg/m2, PO, BID on day1-14, 3 weeks per cycle;

Trastuzumab: The starting dose of 8 mg/kg for IV drip on day1, followed by 6 mg/kg infused once every 3 weeks. The initial infusion lasts about 90 minutes, and subsequently may be shortened to 30-60 minutes if the drug is well tolerated in patients. In the case of treatment delay or interruption, a maintenance dose (6 mg/kg) will be used directly if it has been delayed not longer than 1 week and the loading dose (8 mg/kg) should be adopted again if longer than 1 week. Every 21 days is a treatment cycle.

Identification method for positive HER2 expression: Among the recently obtained results of the pathological examination or review of primary or metastatic lesions by the pathology department of the study sites, the tumor cell immunohistochemical (IHC) staining is shown at least once to be 3+, or the tumor IHC staining is shown to be 2+ and fluorescence in situ hybridization (FISH) or immunochromogenic in situ hybridization.

### Sample size

Based on historical data, the ORR for patients with locally advanced or metastatic advanced gastroesophageal junctions or gastric adenocarcinoma who are previously untreated with systemic therapy is estimated at 45% in first-line chemotherapy. It is assumed that the ORR will be higher than 45% in patients with TTFields concomitant with chemotherapy. The sample size is determined according to the estimated precision, e.g., width of 95% confidence interval because it is an efficacy estimation study. Each of different ORRs falls within the 95% CI range shown below. Hence, at least 45 patients need to be enrolled to ensure that the lower bound of at least 30% of the 95% CI. However, in consideration of a drop-out rate of 10%, 50 patients will be actually enrolled.

**Table 33**

| N=45 | | |
|---|---|---|
| # of responders | ORR | 95% CI |
| 20 | 20/45=44% | (0.30, 0.60) |
| 23 | 23/45=51% | (0.36, 0.66) |
| 25 | 25/45=56% | (0.40, 0.70) |
| 27 | 27/45=60% | (0.44, 0.74) |
| 29 | 29/45=64% | (0.49, 0.78) |

### Analysis set

Intent-To-Treat (ITT) - All patients who sign the Informed Consent Form and are included in the clinical trial.

Full Analysis Set (FAS) - All patients who have received at least one dose of the investigational drug and have measurable tumors at baseline.

Per-Protocol Set (PPS) ― All patients with at least one post-treatment imaging assessment of tumors, good compliance, and no serious violation or deviation against the study protocol in FAS population.

Safety Assessment Set (SS) - All patients who have received at least one dose of the investigational drug and undergone safety assessment.

### Efficacy analysis

Primary efficacy endpoint:
The primary efficacy endpoint is the investigator-assessed ORR.

Objective Response Rate (ORR): The proportion of patients who receive the study treatment and have the best overall response (BOR) rated as per RECIST

1.1 as complete response (CR) or partial response (PR).

### Secondary efficacy endpoints

Kaplan-Meier method is used to estimate the survival functions for time to tumor progression (TTP), progression-free survival (PFS), and overall survival (OS), respectively, and their survival curves plots, the median TTP, median PFS, 12-month overall survival rate and their respective 95% confidence intervals (95% CI) will be provided. Please refer to the statistical analysis plan for details.

Note that while certain examples describe an *in vitro* studies, the results of that study can be extended to the *in vivo* context by performing the administering and the applying to living subjects (e.g. using the Optune^{®} system) instead of to cancer cells *in vitro.*

Note that in the *in vitro* context, the delivery of the various drugs discussed in certain examples to the cancer cells occurs continuously from a first time (t₁) when the drug or drugs were introduced into the container that is holding the cancer cells until such time (t₂) as the drug or drugs were removed or exhausted. As a result, if TTFields are applied to the cancer cells between t₁ and t₂, the applying step will be simultaneous with at least a portion of the delivering step. In the *in vivo* context, the delivery of the drug or drugs to the cancer cells can occur continuously from a first time (t₁) when the drug or drugs are circulating in the patient's body (e.g., after administering it systemically) or introduced into the vicinity of the cancer cells until such time (t₂) as the drug or drugs are eliminated from the patient's body or exhausted. As a result, if TTFields are applied to the cancer cells between t₁ and t₂, the applying step will be simultaneous with at least a portion of the delivering step.

While the present invention has been disclosed with reference to certain embodiments, numerous modifications, alterations, and changes to the described embodiments are possible without departing from the sphere and scope of the present invention, as defined in the appended claims. Accordingly, it is intended that the present invention not be limited to the described embodiments, but that it has the full scope defined by the language of the following claims, and equivalents thereof.

### STATEMENTS

Furthermore, the present invention is represented by the following statements of invention.
1. A method of treating gastric cancer in a subject, the method comprising:
   administering oxaliplatin and capecitabine to the subject; and
   applying an alternating electric field to a target region of the subject, the alternating electric field having a frequency between 100 and 400 kHz.
2. The method of statement 1, wherein at least a portion of the applying is performed after the administering and before the oxaliplatin and capecitabine are eliminated from the subject's body.
3. The method of statement 1, wherein the applying has a duration of at least 72 hours.
4. The method of statement 1, wherein the frequency of the alternating electric field is between 120 and 180 kHz.
5. The method of statement 1, wherein the frequency of the alternating electric field is between 145 and 155 kHz.
6. The method of statement 1, wherein the oxaliplatin and capecitabine are administered at therapeutically effective doses, and wherein the alternating electric field has a field strength of at least 1 V/cm in at least a portion of the target region.
7. The method of statement 6, wherein the applying has a duration of at least 72 hours and wherein the frequency of the alternating electric field is between 120 and 180 kHz.
8. The method of statement 6, wherein the applying has a duration of at least 72 hours and wherein the frequency of the alternating electric field is between 145 and 155 kHz.
9. A method of reducing viability of gastric cancer cells, the method comprising:
   administering oxaliplatin and capecitabine to the cancer cells; and
   applying an alternating electric field to the cancer cells, the alternating electric field having a frequency between 100 and 400 kHz.
10. The method of statement 9, wherein at least a portion of the applying is performed simultaneously with at least a portion of the administering.
11. The method of statement 9, wherein the applying has a duration of at least 72 hours.
12. The method of statement 9, wherein the frequency of the alternating electric field is between 120 and 180 kHz.
13. The method of statement 9, wherein the frequency of the alternating electric field is between 145 and 155 kHz.
14. The method of statement 9, wherein each of the oxaliplatin and capecitabine is delivered to the cancer cells at a therapeutically effective concentration, and wherein the alternating electric field has a field strength of at least 1 V/cm in at least some of the cancer cells.
15. The method of statement 14, wherein the applying has a duration of at least 72 hours and wherein the frequency of the alternating electric field is between 120 and 180 kHz.
16. The method of statement 14, wherein the applying has a duration of at least 72 hours and wherein the frequency of the alternating electric field is between 145 and 155 kHz.
17. A method of treating gastric cancer in a subject, the method comprising:
   administering folinic acid, fluorouracil, and oxaliplatin to the subject; and
   applying an alternating electric field to a target region of the subject, the alternating electric field having a frequency between 100 and 400 kHz.
18. The method of statement 17, wherein at least a portion of the applying is performed after the administering and before the folinic acid, fluorouracil, and oxaliplatin are eliminated from the subject's body.
19. The method of statement 17, wherein the applying has a duration of at least 72 hours.
20. The method of statement 17, wherein the frequency of the alternating electric field is between 120 and 180 kHz.
21. The method of statement 17, wherein the frequency of the alternating electric field is between 145 and 155 kHz.
22. The method of statement 17, wherein the folinic acid, fluorouracil, and oxaliplatin are administered at therapeutically effective doses, and wherein the alternating electric field has a field strength of at least 1 V/cm in at least a portion of the target region.
23. The method of statement 22, wherein the applying has a duration of at least 72 hours and wherein the frequency of the alternating electric field is between 120 and 180 kHz.
24. The method of statement 22, wherein the applying has a duration of at least 72 hours and wherein the frequency of the alternating electric field is between 145 and 155 kHz.
25. A method of reducing viability of gastric cancer cells, the method comprising:
   administering folinic acid, fluorouracil, and oxaliplatin to the cancer cells; and
   applying an alternating electric field to the cancer cells, the alternating electric field having a frequency between 100 and 400 kHz.
26. The method of statement 25, wherein at least a portion of the applying is performed simultaneously with at least a portion of the administering.
27. The method of statement 25, wherein the applying has a duration of at least 72 hours.
28. The method of statement 25, wherein the frequency of the alternating electric field is between 120 and 180 kHz.
29. The method of statement 25, wherein the frequency of the alternating electric field is between 145 and 155 kHz.
30. The method of statement 25, wherein each of the folinic acid, fluorouracil, and oxaliplatin is delivered to the cancer cells at a therapeutically effective concentration, and wherein the alternating electric field has a field strength of at least 1 V/cm in at least some of the cancer cells.
31. The method of statement 30, wherein the applying has a duration of at least 72 hours and wherein the frequency of the alternating electric field is between 120 and 180 kHz.
32. The method of statement 30, wherein the applying has a duration of at least 72 hours and wherein the frequency of the alternating electric field is between 145 and 155 kHz.
33. A method of treating gastric cancer in a subject, the method comprising:
   administering fluorouracil to the subject; and
   applying an alternating electric field to a target region of the subject, the alternating electric field having a frequency between 100 and 400 kHz.
34. The method of statement 33, wherein at least a portion of the applying is performed after the administering and before the fluorouracil is eliminated from the subject's body or exhausted.
35. The method of statement 33, wherein the applying has a duration of at least 72 hours.
36. The method of statement 33, wherein the frequency of the alternating electric field is between 120 and 180 kHz.
37. The method of statement 33, wherein the frequency of the alternating electric field is between 145 and 155 kHz.
38. The method of statement 33, wherein the fluorouracil is administered at therapeutically effective dose, and wherein the alternating electric field has a field strength of at least 1 V/cm in at least a portion of the target region.
39. The method of statement 33, wherein the administration of the fluorouracil is accomplished by direct administration of the fluorouracil to the subject.
40. The method of statement 33, wherein the administration of the fluorouracil is accomplished by indirect administration of the fluorouracil to the subject.
41. The method of statement 40, wherein the indirect administration of the fluorouracil is accomplished by administering a pre-prodrug to the subject, wherein the pre-prodrug is converted to fluorouracil in the subject's body.
42. The method of statement 41, wherein the applying has a duration of at least 72 hours and wherein the frequency of the alternating electric field is between 120 and 180 kHz.
43. The method of statement 41, wherein the applying has a duration of at least 72 hours and wherein the frequency of the alternating electric field is between 145 and 155 kHz.
44. A method of reducing viability of gastric cancer cells, the method comprising:
   administering fluorouracil to the cancer cells; and
   applying an alternating electric field to the cancer cells, the alternating electric field having a frequency between 100 and 400 kHz.
45. The method of statement 44, wherein at least a portion of the applying is performed simultaneously with at least a portion of the administering.
46. The method of statement 44, wherein the applying has a duration of at least 72 hours.
47. The method of statement 44, wherein the frequency of the alternating electric field is between 120 and 180 kHz.
48. The method of statement 44, wherein the frequency of the alternating electric field is between 145 and 155 kHz.
49. The method of statement 44, wherein the fluorouracil is administered to the cancer cells at a therapeutically effective concentration, and wherein the alternating electric field has a field strength of at least 1 V/cm in at least some of the cancer cells.
50. The method of statement 49, wherein the applying has a duration of at least 72 hours and wherein the frequency of the alternating electric field is between 120 and 180 kHz.
51. The method of statement 49, wherein the applying has a duration of at least 72 hours and wherein the frequency of the alternating electric field is between 145 and 155 kHz.
52. A method of reducing viability of gastric cancer cells in a subject, the method comprising:
   administering fluorouracil to the subject; and
   applying an alternating electric field to the gastric cancer cells in the subject, the alternating electric field having a frequency between 100 and 400 kHz.
53. The method of statement 52, wherein the administration of the fluorouracil is accomplished by direct administration of the fluorouracil to the subject.
54. The method of statement 52, wherein the administration of the fluorouracil is accomplished by indirect administration of the fluorouracil to the subject.
55. The method of statement 54, wherein the indirect administration of the fluorouracil is accomplished by administering a pre-prodrug to the subject, wherein the pre-prodrug is converted to fluorouracil in the subject's body.

## Claims

1. Fluorouracil, as drug, for use in a method of treating gastric cancer or reducing viability of gastric cancer cells in a subject or reducing viability of gastric cancer cells, the method comprising administering the fluorouracil to the subject or the cancer cells and applying an alternating electric field to a target region of the subject or the cancer cells, the alternating electric field having a frequency between 100 and 400 kHz.

2. The drug of claim 1, wherein at least a portion of the applying is performed after the administering and before the fluorouracil is eliminated from the subject's body or exhausted.

3. The drug of claim 1, wherein the applying has a duration of at least 72 hours.

4. The drug of claim 1, wherein the frequency of the alternating electric field is between 120 and 180 kHz.

5. The drug of claim 1, wherein the frequency of the alternating electric field is between 145 and 155 kHz.

6. The drug of claim 1, wherein the fluorouracil is administered at a therapeutically effective dose, and the alternating electric field has a field strength of at least 1 V/cm in at least a portion of the target region.

7. The drug of claim 1, wherein the administration of the fluorouracil is accomplished by direct administration of the fluorouracil to the subject.

8. The drug of claim 1, wherein the administration of the fluorouracil is accomplished by indirect administration of the fluorouracil to the subject.

9. The drug of claim 8, wherein the indirect administration of the fluorouracil is accomplished by administering a pre-prodrug to the subject, the pre-prodrug being converted to fluorouracil in the subject's body.

10. The drug of claim 9, wherein the applying has a duration of at least 72 hours and the frequency of the alternating electric field is between 120 and 180 kHz.

11. The drug of claim 9, wherein the applying has a duration of at least 72 hours and the frequency of the alternating electric field is between 145 and 155 kHz.

12. The drug of claim 1, wherein at least a portion of the applying is performed simultaneously with at least a portion of the administering.

13. The drug of claim 1, wherein the fluorouracil is administered to the cancer cells at a therapeutically effective concentration, and the alternating electric field has a field strength of at least 1 V/cm in at least some of the cancer cells.

14. The drug of claim 13, wherein the applying has a duration of at least 72 hours and the frequency of the alternating electric field is between 120 and 180 kHz.

15. The drug of claim 13, wherein the applying has a duration of at least 72 hours and the frequency of the alternating electric field is between 145 and 155 kHz.
